(19) Europäisches Patentamt / European Patent Office / Office européen des brevets

(11) **EP 2 325 202 B1**

(12) **EUROPEAN PATENT SPECIFICATION**

(45) Date of publication and mention
of the grant of the patent:
**22.10.2014 Bulletin 2014/43**

(51) Int Cl.:
**C07K 14/56** (2006.01)

(21) Application number: **10193126.9**

(22) Date of filing: **26.08.2004**

(54) **Uses of interferons with altered spatial structure**

Verwendungen von Interferonen mit veränderter Raumstruktur

Utilisations d'interférons à structure spatiale modifiée

(84) Designated Contracting States:
**AT BE BG CH CY CZ DE DK EE ES FI FR GB GR HU IE IT LI LU MC NL PL PT RO SE SI SK TR**

(30) Priority: **28.08.2003 US 498923 P
28.08.2003 US 498449 P
28.08.2003 US 498785 P
05.03.2004 IN MU02802004
05.03.2004 IN MU02792004**

(43) Date of publication of application:
**25.05.2011 Bulletin 2011/21**

(62) Document number(s) of the earlier application(s) in
accordance with Art. 76 EPC:
**04809634.1 / 1 663 110**

(73) Proprietor: **Superlab Far East Limited
Road Town, Tortola (VG)**

(72) Inventor: **Wei, Guangwen
610 017 Chengdu (CN)**

(74) Representative: **Capasso, Olga et al
De Simone & Partners S.p.A.
Via Vincenzo Bellini, 20
00198 Roma (IT)**

(56) References cited:
**WO-A1-02/080958      AU-A1- 2003 248 419
US-A- 4 695 623**

- DATABASE MEDLINE [Online] US NATIONAL LIBRARY OF MEDICINE (NLM), BETHESDA, MD, US; June 2003 (2003-06), GAO ZHAN-CHENG ET AL: "[Clinical investigation of outbreak of nosocomial severe acute respiratory syndrome]", XP002294985, Database accession no. NLM12837162 & ZHONGGUO WEI ZHONG BING JI JIU YI XUE = CHINESE CRITICAL CARE MEDICINE = ZHONGGUO WEIZHONGBING JIJIUYIXUE JUN 2003, vol. 15, no. 6, June 2003 (2003-06), pages 332-335, ISSN: 1003-0603
- ZHAO Z ET AL: "DESCRIPTION AND CLINICAL TREATMENT OF AN EARLY OUTBREAK OF SEVERE ACUTE RESPIRATORY SYNDROME (SARS) IN GUANGZHOU PR CHINA", JOURNAL OF MEDICAL MICROBIOLOGY, HARLOW, GB, vol. 52, no. 8, 1 August 2003 (2003-08-01) , pages 715-720, XP009035954, ISSN: 0022-2615
- BLATT L M ET AL: "THE BIOLOGIC ACTIVITY AND MOLECULAR CHARACTERIZATION OF A NOVEL SYNTHETIC INTERFERON-ALPHA SPECIES, CONSENSUS INTERFERON", JOURNAL OF INTERFERON AND CYTOKINE RESEARCH, MARY ANN LIEBERT, NEW YORK, NY, US, vol. 16, no. 7, 1 January 1996 (1996-01-01), pages 489-499, XP009023878, ISSN: 1079-9907
- HIGGINS P G ET AL: "INTRANASAL INTERFERON AS PROTECTION AGAINST EXPERIMENTAL RESPIRATORY CORONAVIRUS INFECTION IN VOLUNTEERS", ANTIMICROBIAL AGENTS AND CHEMOTHERAPY, AMERICAN SOCIETY FOR MICROBIOLOGY, WASHINGTON, DC, US, vol. 24, no. 5, 1 November 1983 (1983-11-01), pages 713-715, XP009016436, ISSN: 0066-4804

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- LOUTFY MONA R ET AL: "Interferon alfacon-1 plus corticosteroids in severe acute respiratory syndrome: A preliminary study.", JAMA (JOURNAL OF THE AMERICAN MEDICAL ASSOCIATION), vol. 290, no. 24, 24 December 2003 (2003-12-24), pages 3222-3228, XP002632357, ISSN: 0098-7484

- SPADA S ET AL: "Infergen", 20040101, 1 January 2004 (2004-01-01), pages 116-117, XP008108470,

**Description**

**FIELD OF THE INVENTION**

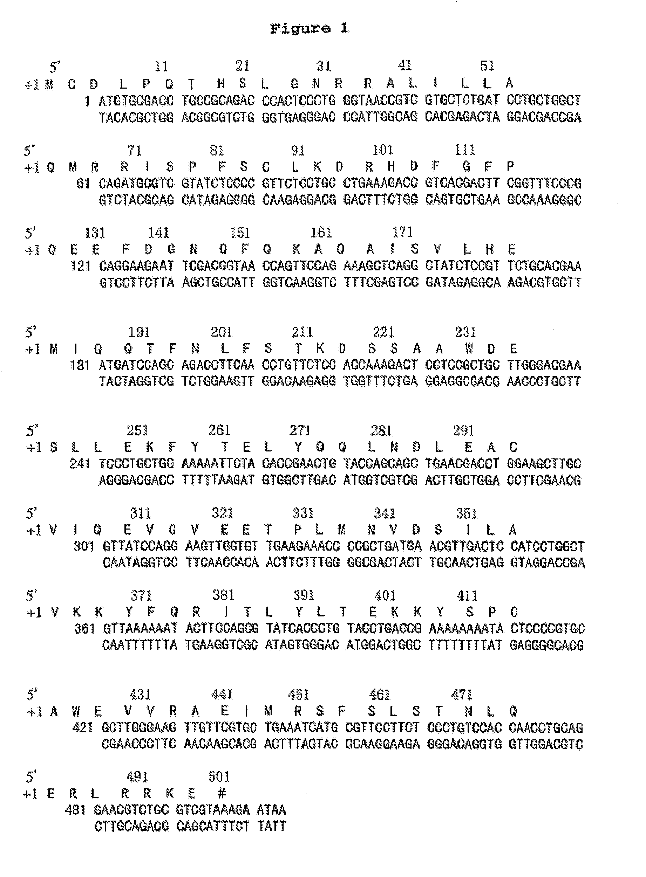

This invention is related to a recombinant super-compound interferon (rSIFN-co) obtainable by a process comprising introducing into E. Coli the polynucleotide sequence as shown in Fig. 1, with changed spatial configuration. One characteristic of rSIFN-co in this invention is that it cannot only inhibit DNA (deoxyribonucleic acid) duplication of the hepatitis B virus but also the secretion of HBsAg and HBeAg.

**BACKGROUND OF THE INVENTION**

rSIFN-co is a new interferon molecule constructed with the most popular conservative amino acid found in natural human α-IFN subtypes using genetic engineering methods. United States Patent Nos. 4,695,623 and 4,897,471 have described it. rSIFN-co had been proven to have broad-spectrum IFN activity and virus- and tumor-inhibition and natural killer cell activity. United States Patent No. 5,372,808 by Amgen, Inc. addresses treatment rSIFN-co. Chinese Patent No. 97193506.8 by Amgen, Inc. addresses re-treatment of rSIFN-co on hepatitis C. Chinese Patent No. 98114663.5 by Shenzhen Jiusheng Bio-engineering Ltd. addresses rSIFN-co treatment for hepatitis B and hepatitis C.
The United States Food and Drug Administration (FDA) authorized Amgen to produce rSIFN-co with *E. Coli.* for clinical hepatitis C treatment at the end of 1997.
Hepatitis B patients can be identified when detecting HBsAg and the HBeAg. α-IFN is commonly used in clinics to treat hepatitis B. IFN binds superficial cell membrane receptors, inhibiting DNA and RNA (ribonucleic acid) duplication, including inducing some enzymes to prevent duplication of the virus in hepatitis-infected cells. All IFNs can inhibit only the DNA duplication of viruses, not the e and s antigen.
This disclosure describes recombinant super-compound interferon, method to produce the same and uses thereof.
An outbreak of atypical pneumonia, referred to as severe acute respiratory syndrome (SARS) and first identified in Guangdong Province, China, has spread to several countries. Similar cases were detected in patients in Hong Kong, Vietnam, and Canada from February and March 2003. The World Health Organization (WHO) issued a global alert for the illness. In mid-March 2003, SARS was recognized in health care workers and household members who had cared for patients with severe respiratory illness in the Far East. Many of these cases could be traced through multiple chains of transmission to a health care worker from Guangdong Province who visited Hong Kong, where he was hospitalized with pneumonia and died. By late April 2003, thousands of SARS cases and hundreds of SARS-related deaths were reported to WHO from over 25 countries around the world. Most of these cases occurred after exposure to SARS patients in household or health care settings. This disclosure provides a method to prevent and/or treat SARS.
Another current epidemic scare in Asia is the avian influenza virus (H5N1). Avian influenza is an infectious disease in birds caused by type A strains of the influenza virus. There are 15 avian influenza virus subtypes; H5N1 is of particular concern because it mutates rapidly infecting not just animals, but humans. The confirmed human death count from avian influenza, as of February 4, 2004, stood at thirteen. Laboratories in the WHO global influenza network have been working to control the virus and prevent further human deaths. However, to fully understand the magnitude of H5N1 and its ways of distribution, more meticulous testing is needed. Furthermore, antiviral drugs are only effective in treating or preventing influenza A virus strains against those who are of fair health. See http://www.who.int/csr/don/2004_01_15/en, January 15, 2004.
Researchers at St. Jude and other top influenza laboratories are racing to create a prototype human vaccine against H5N1. They hope that prototype vaccines can be ready in as little as three weeks. Nevertheless, until a vaccine is created, scientists are worried that H5N1 may develop into a human superflu. See The Wall Street Journal, Scientists Rush to Create Vaccine for Bird Flu - Just in Case, January 28, 2004.
This disclosure describes recombinant super-compound interferon **as defined above**, method to produce the same and uses thereof. Particularly, the super-compound interferon disclosed herein is capable of inhibiting, preventing and/or treating the hepatitis viruses, SARS virus, or virus-induced upper respiratory diseases, and the avian influenza virus.

**SUMMARY OF THE INVENTION**

This invention provides a method for inhibiting, preventing or treating viral diseases or tumors in a subject comprising administering to the subject an effective amount of the super-compound interferon **obtainable by a process comprising introducing into E. Coli the polynucleotide sequence as shown in Fig. 1.**
This invention provides the above-described method wherein super-compound interferon is administered orally via vein injection, muscle injection, peritoneal injection, subcutaneous injection, nasal or mucosal administration, or by inhalation via an inspirator.

This invention provides the method to prevent or treat viral diseases wherein the viral diseases is hepatitis A, hepatitis B, hepatitis C, other types of hepatitis, infections of viruses caused by Epstein-Barr virus, Cytomegalovirus, herpes simplex viruses, or other types of herpes viruses, papovaviruses, poxviruses, picornaviruses, adenoviruses, rhinoviruses, human T-cell leukemia viruses I, or human T-cell leukemia viruses II, or human T-cell leukemia virus III.

This invention provides a method for anti-hepatitis activities. It can inhibit HBV-DNA replication, HBsAg and HBeAg production.

This invention provides a method to prevent or treat upper respiratory infection diseases.

This invention provides a method to prevent or treat tumors or cancers wherein the tumor is skin cancer, basal cell carcinoma and malignant melanoma, renal cell carcinoma, liver cancer, thyroid cancer, rhinopharyngeal cancer, solid carcinoma, prostate cancer, stomach/abdominal cancer, esophageal cancer, rectal cancer, pancreatic cancer, breast cancer, ovarian cancer, and superficial bladder cancer, hemangioma, epidermoid carcinoma, cervical cancer, non-small-cell lung cancer, small-cell lung cancer, glioma, leucocythemia, acute leucocythemia and chronic leucocythemia, chronica myelocytic leukemia, hairy cell leukemia, lymphadenoma, multiple myeloma, polycythemia vera, or Kaposi's sarcoma.

This invention provides a method for preventing or treating Severe Acute Respiratory Syndrome (SARS) or virus-induced upper respiratory diseases in a subject comprising administering to the subject an effective amount of recombinant super-compound interferon **as defined above**.

The super-compound interferon may be administered orally, via vein injection, muscle injection, peritoneal injection, subcutaneous injection, nasal or mucosal administration, or by inhalation via an inspirator.

This invention provides a method for inhibiting the causative agent of Severe Acute Respiratory Syndrome, or virus-induced upper respiratory diseases, comprising contacting the agent with an effective amount of super-compound interferon.

This invention also provides a method for inhibiting Severe Acute Respiratory Syndrome virus, Severe Acute Respiratory Syndrome virus-infected cells, or virus-induced upper respiratory diseases, comprising contacting an effective amount of the super-compound interferon with said virus or cells. This contact could be direct or indirect.

This invention provides a composition comprising an effective amount of the super-compound interferon capable of inhibiting, preventing or treating Severe Acute Respiratory Syndrome virus, Severe Acute Respiratory Syndrome virus-infected cells, or virus-induced upper respiratory diseases, and a suitable carrier.

This invention provides a pharmaceutical composition comprising an effective amount of the recombinant super-compound interferon **as defined above** capable of inhibiting, preventing or treating Severe Acute Respiratory Syndrome, Severe Acute Respiratory Syndrome virus-infected cells, or virus-induced upper respiratory diseases in a subject, and a pharmaceutically acceptable carrier.

It is an object of the invention a method for preventing or treating Severe Acute Respiratory Syndrome in a subject comprising administering to the subject an effective amount of recombinant super-compound interferon **as defined above** thereof.

It is an object of the invention a recombinant interferon obtainable by a process comprising introducing into E. coli the polynucleotide sequence shown in Figure 1, for use as a medicament for treating a viral disease in a subject.

It is a further object of the invention pharmaceutical composition comprising a recombinant interferon obtainable by a process comprising introducing into E. coli the polynucleotide sequence shown in Figure 1 and a pharmaceutically acceptable carrier, for use in the treatment of a viral disease.

Preferably the viral disease is hepatitis A, hepatitis B, hepatitis C, other types of hepatitis, or virus-induced upper respiratory disease. More preferably the viral disease is an infection caused by Epstein-Barr virus, Cytomegalovirus, herpes simplex virus, other types of herpes virus, papovavirus, poxvirus, picornavirus, adenovirus, rhinovirus, human T-cell leukemia virus I, human T-cell leukemia virus II, human T-cell leukemia virus III, or avian influenza virus.

It is a further object of the invention a recombinant interferon obtainable by a process comprising introducing into E. coli the polynucleotide sequence shown in Figure 1, for use as a medicament for treating tumor in a subject.

It is a further object of the invention a pharmaceutical composition comprising a recombinant interferon obtainable by a process comprising introducing into E. coli the polynucleotide sequence shown in Figure 1 and a pharmaceutically acceptable carrier, for use in the treatment of a tumor.

Preferably the tumor is skin cancer, basal cell carcinoma, malignant melanoma, liver cancer, renal cell carcinoma, thyroid cancer, rhinopharyngeal cancer, solid carcinoma, prostate cancer, stomach cancer, abdominal cancer, esophageal cancer, rectal cancer, pancreatic cancer, breast cancer, ovarian cancer, superficial bladder cancer, hemangioma, cervical cancer, non-small cell lung cancer, small cell lung cancer, glioma, acute leucocythemia, chronic leucocythemia, chronic myelocytic leukemia, hairy cell leukemia, lymphadenoma, multiple myeloma, polycythemia vera, or Kaposi's sarcoma.

Preferably the interferon is administered via oral administration, vein injection, muscle injection, peritoneal injection, subcutaneous injection, nasal administration, mucosal administration, or by inhalation.

Still preferably the recombinant interferon is formulated as tablet, capsule, oral liquid, paste, injection, spray, suppository, or solution.

Preferably the interferon is $\alpha$, $\beta$, or $\omega$. Yet preferably, the interferon is lyophilized.

Preferably, the super-compound interferon is administered orally, via vein injection, muscle injection, peritoneal injection, subcutaneous injection, nasal or mucosal administration, or by inhalation via an inspirator.

Still preferably, the interferon is delivered by a spray device.

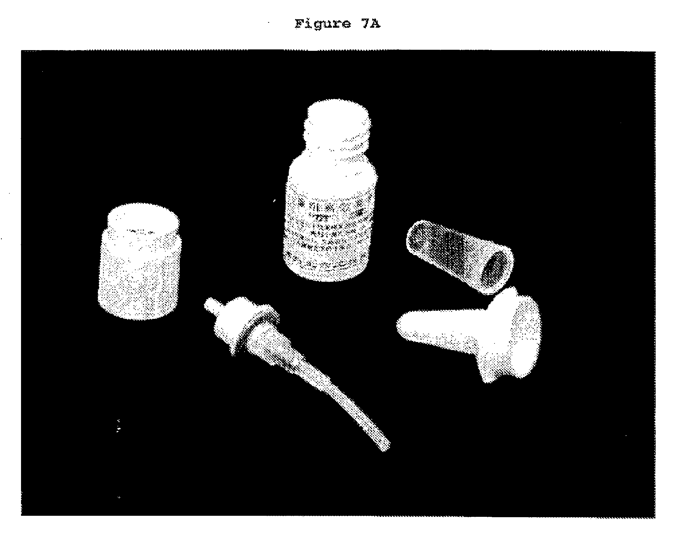

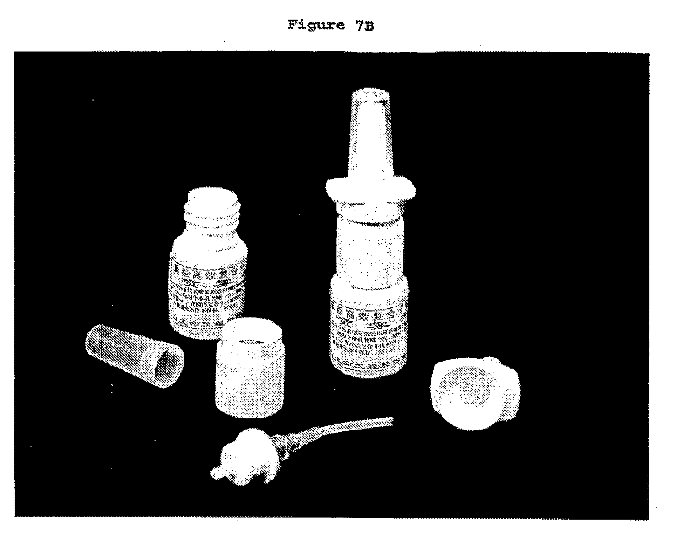

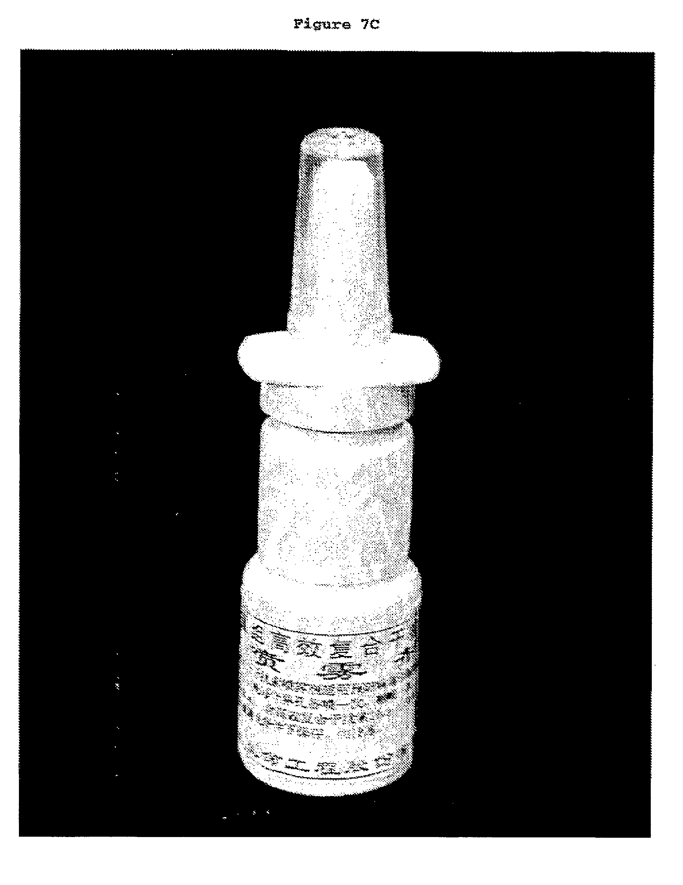

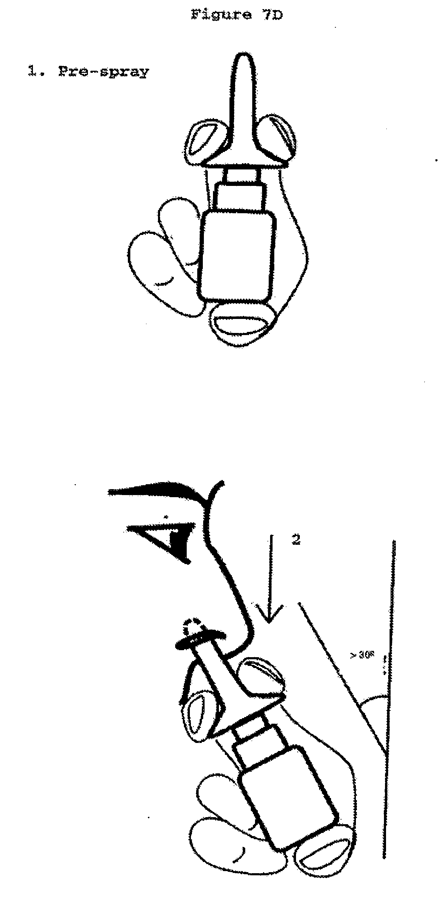

Still preferably, the device is described in Figure 7.

It is a further object of the invention a method for inhibiting the causative agent of Severe Acute Respiratory Syndrome comprising directly or indirectly contacting the agent with an effective amount of super-compound interferon as defined above.

Preferably, the causative agent is a virus.

It is a further object of the invention a method for inhibiting Severe Acute Respiratory Syndrome virus or Severe Acute Respiratory Syndrome virus-infected cells comprising contact an effective amount of the super-compound interferon with said virus or cells.

It is a further object of the invention a composition comprising an effective amount of the super-compound interferon capable of inhibiting Severe Acute Respiratory Syndrome virus or Severe Acute Respiratory Syndrome virus-infected cells and a suitable carrier.

It is a further object of the invention a composition comprising an effective amount of the super-compound interferon capable of preventing or treating Severe Acute Respiratory Syndrome of a subject and a suitable carrier.

It is a further object of the invention a pharmaceutical composition comprising an effective amount of the recombinant super-compound interferon capable of inhibiting Severe Acute Respiratory Syndrome virus or Severe Acute Respiratory Syndrome virus-infected cells and a pharmaceutically acceptable carrier.

It is a further object of the invention a pharmaceutical composition comprising an effective amount of the recombinant super-compound interferon capable of preventing or treating Severe Acute Respiratory Syndrome in a subject and a pharmaceutically acceptable carrier.

It is a further object of the invention a device to deliver the pharmaceutical composition as described above.

Preferably, the subject is a human.

It is a further object of the invention a method for preventing Severe Acute Respiratory Syndrome in humans comprising application of the super-compound interferon three times a day via a spray which contains twenty microgram with ten million units of activity in three milliliter.

## DETAILED DESCRIPTION OF THE FIGURES

**Figure 1.** rSIFN-co cDNA sequence designed according to *E. Coli.* codon usage and deduced rSIFN-co amino acid sequence

**Figure 2.** Sequence of another super-compound interferon

**Figure 3.** Diagram of pLac T7 cloning vector plasmid

**Figure 4.** Diagram of pHY-4 expression vector plasmid

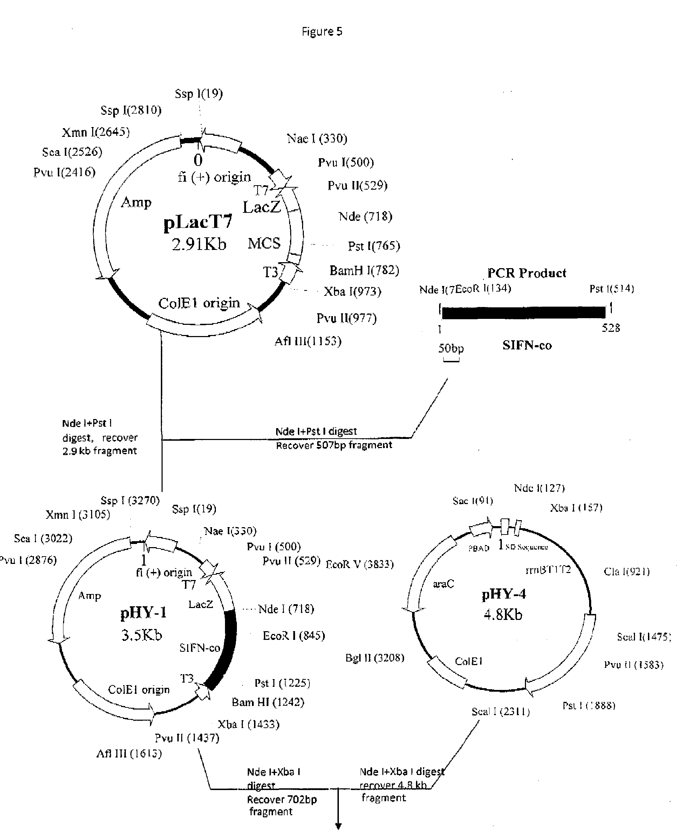

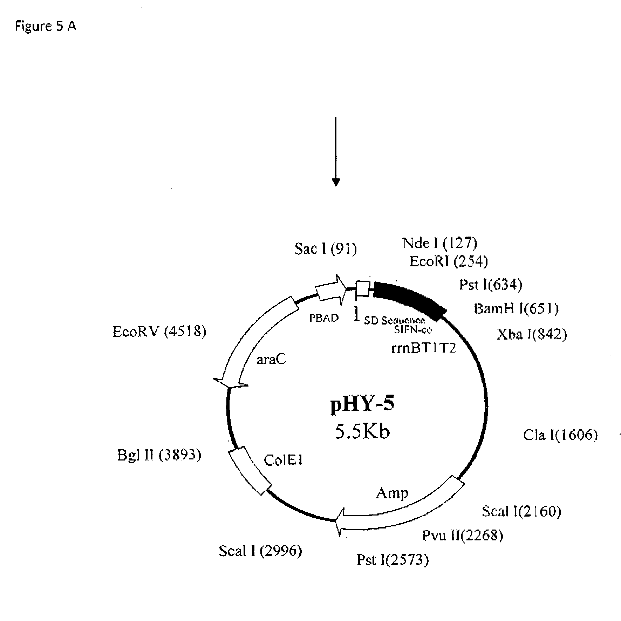

**Figure 5.** Construction process of expression plasmid pHY-5

**Figure 6-A**. Circular Dichroism spectrum of Infergen®
(Tested by Analysis and Measurement Center of Sichuan University)
Spectrum range: 250nm - 190nm
Sensitivity: 2 m°/cm
Light path: 0.20 cm
Equipment: Circular Dichroism J-500C
Samples: contains $30\mu$g/ml IFN-con1, 5.9 mg/ml of NaCl and 3.8 mg/ml of $Na_2PO_4$ pH7.0.
Infergen® (interferon alfacon-1), made by Amgen Inc., also known as consensus interferon, is marketed for the treatment of adults with chronic hepatitis C virus (HCV) infections. It is currently the only FDA-approved, bio-optimized interferon developed through rational drug design and the only interferon with data on the label specifically for non-responding or refractory patients. InterMune's sales force re-launched Infergen® in January 2002 with an active campaign to educate U.S. hepatologists about the safe and appropriate use of Infergen®, which represents new hope for the more than 50 percent of HCV patients who fail other currently available therapies. See http://www.intermune.com/wt/itmn/infergen, 8/27/2003

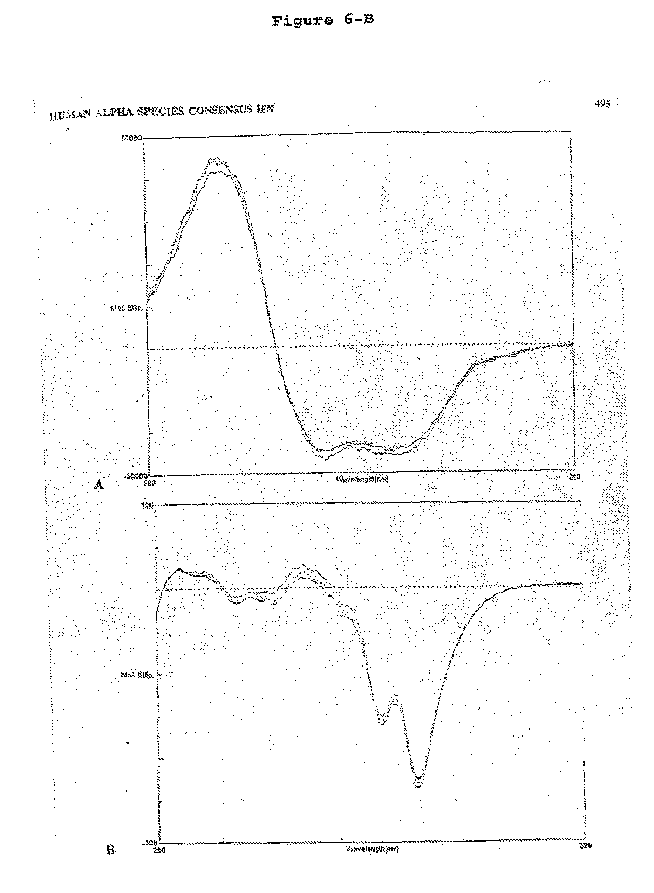

**Figure 6-B**. Circular Dichroism spectrum of Infergen® From Reference [Journal of Interferon and Cytokine Research. 16:489-499(1996)]
Circular dichroism spectra of concensus interferon subforms. Concensus interferon was fractionated using an anion exchange column. Samples were dialyzed into 10mM sodium phosphate, pH 7.4. Measurements were made on Jasco J-170 spectopolarimeter, in a cell thermostat at 15°C. (———), acylated form; (--) cis terminal form; (...), met terminal form. A. Far UV Spectrum. B. Near UV Spectrum.

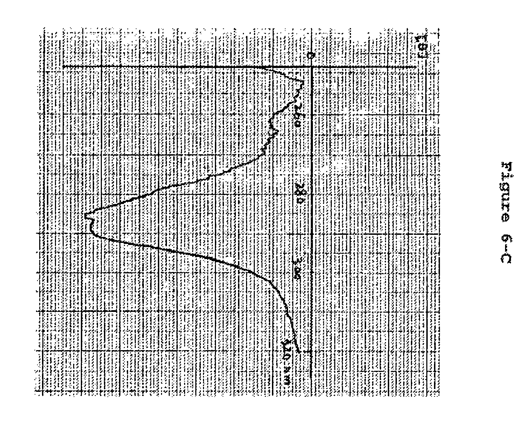

**Figure 6-C**. Circular Dichroism spectrum of rSIFN-co Spectrum range: 320nm-250nm
Sensitivity: 2 m°/cm
Light path: 2cm
Equipment: Circular Dichroism J-500C
Samples: contains 0.5mg/ml rSIFN-co, 5.9 mg/ml of NaCl and 3.8 mg/ml of $Na_2PO_4$ pH7.0.

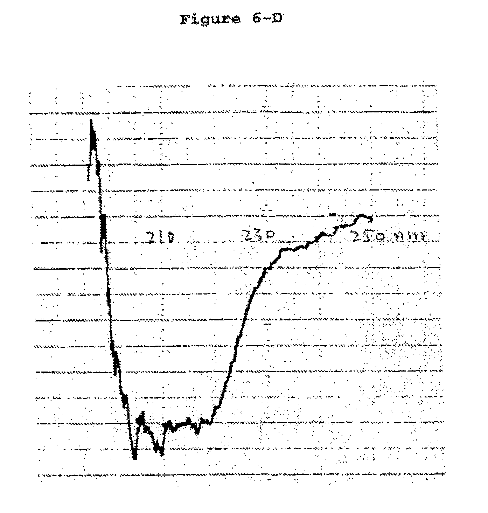

**Figure 6-D**. Circular Dichroism spectrum of rSIFN-co Spectrum range: 250nm - 190nm
Sensitivity: 2 m°/cm
Light path: 0.20 cm
Equipment: Circular Dichroism J-500C
Samples: contains 30pg/ml rSIFN-co, 5.9 mg/ml of NaCl and 3.8 mg/ml of $Na_2PO_4$ pH7.0.
Clearly, as evidenced by the above spectra, the secondary or even tertiary structure of rSIFN-co is different from Infergen®.

**Figure 7A-C**. Recombinant Super-Compound Interferon Spray Height: 90 mm
Width: 25mm (bottom), 6mm (top)
Weight: 9g
Volume delivery: 0.1 ml

**Figure 7D.** Recombinant Super-Compound Interferon Spray
When using the spray for the first time, take off the cap and discharge in the air several times until some liquid squirts out. Do not need to test spray for subsequent uses. To use, follow the illustrations shown in the figure, i.e.: (1) Pre-spray and (2) Press down on the nozzle to release the medication.

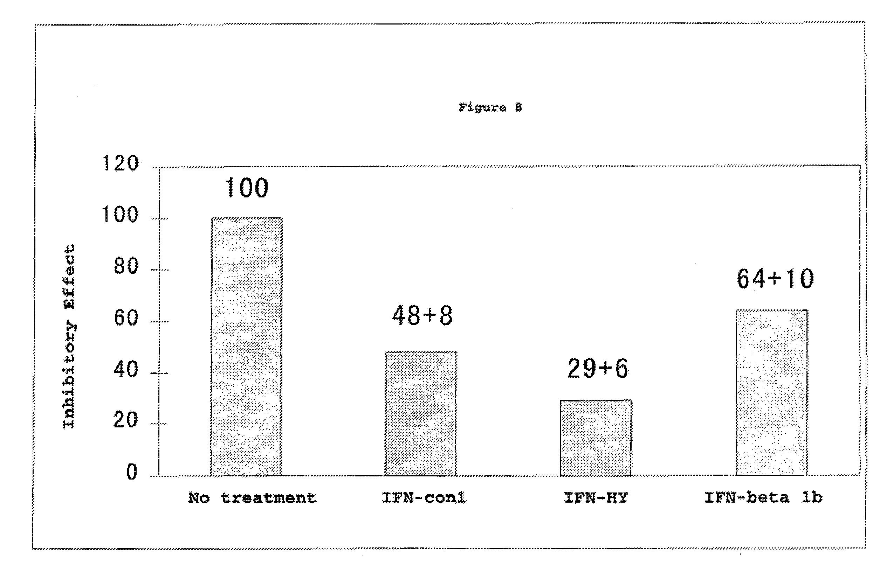

**Figure 8.** Comparison of Inhibition Effects of Different Interferons on HBV Gene Expression

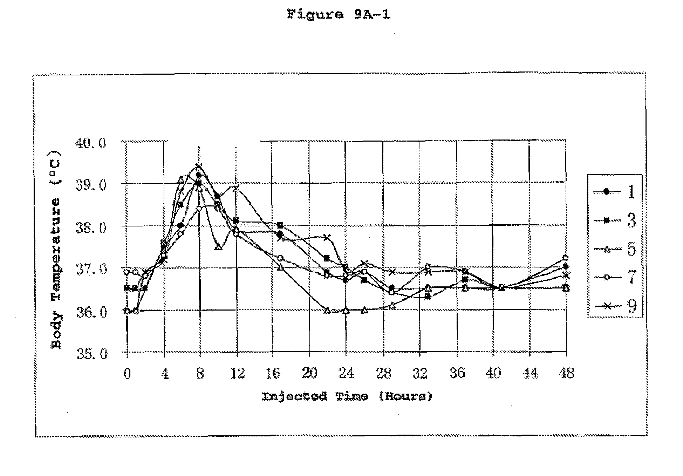

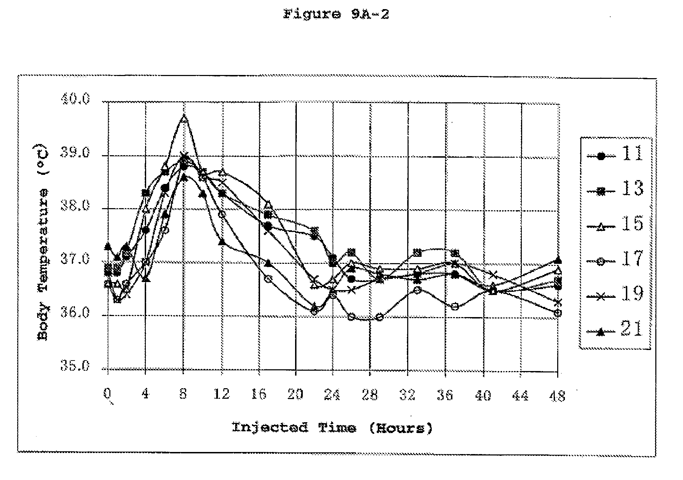

**Figure 9A-1.** Curves of Changes of Body Temperature in Group A (5 patients)
This figure is the record of body temperature changes of 5 patients in Group A.

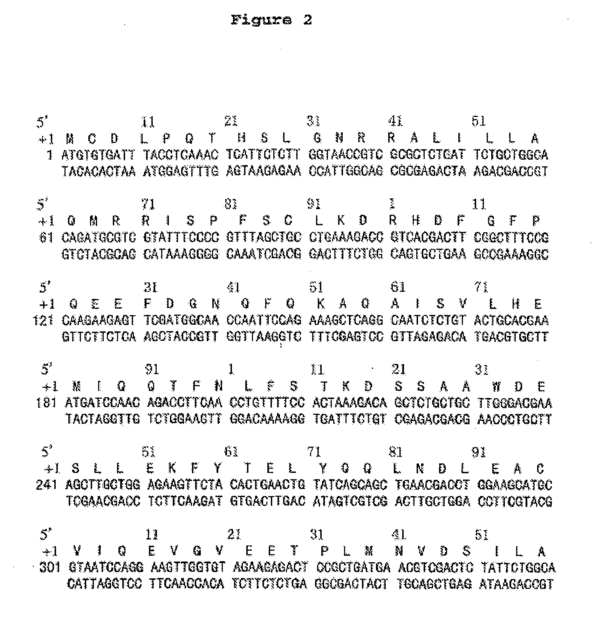

**Figure 9A-2**. Curves of Changes of Body Temperature in Group A (6 patients)
This figure is the record of body temperature changes of the other 6 patients in Group A.

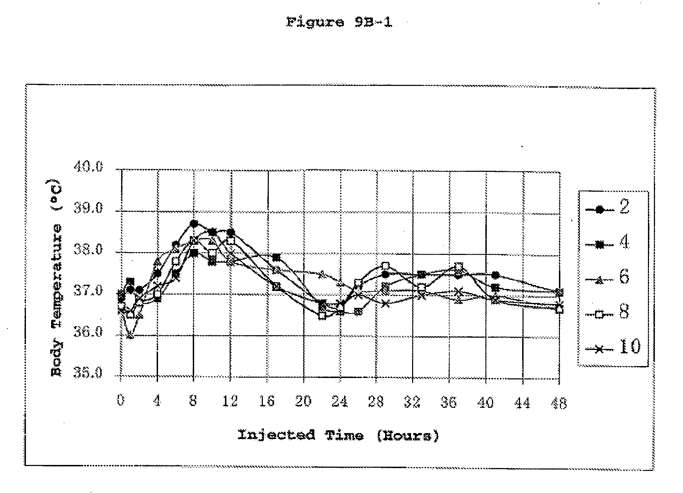

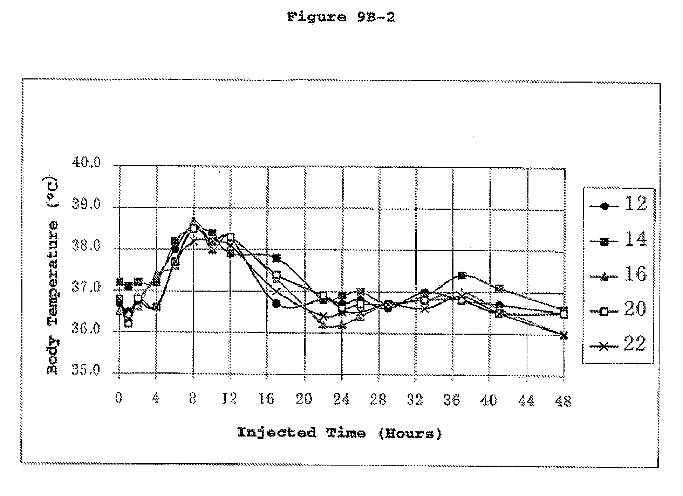

**Figure 9B-1**. Curves of Changes of Body Temperature in Group B (5 patients)
This figure is the record of body temperature changes of 5 patients in Group B.

**Figure 9B-2**. Curves of Changes of Body Temperature in Group B (5 patients)
This figure is the record of body temperature changes of the other 5 patients in Group B.

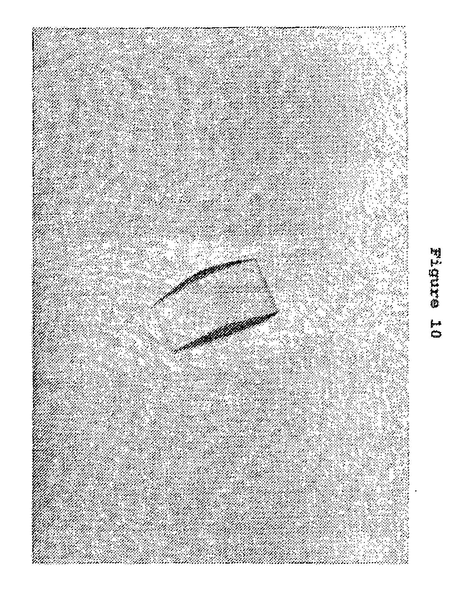

**Figure 10.** rsIFN-co Crystal I

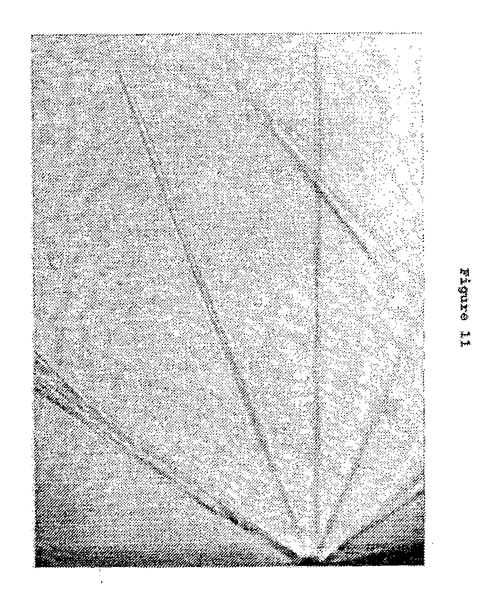

**Figure 11.** rsIFN-co Crystal II

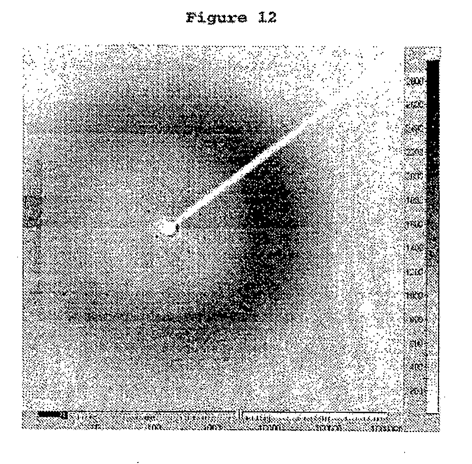

**Figure 12.** The X-ray Diffraction of rsIFN-co Crystal

## DETAILED DESCRIPTION OF THE INVENTION

This invention provides a method for producing a recombinant super-compound interferon with changed spatial configuration and enhanced antiviral activity comprising steps of:

(a) Introducing nucleic acid molecule **as shown in Fig.1** which codes for said interferon with preferred codons for expression to an **E. Coli** host; and

(b) Placing the introduced host in conditions allowing expression of said interferon.

This invention provides the method for producing interferon, further comprising recovery of the expressed interferon. This invention provides a recombinant super-compound interferon as described above with changed spatial configuration. This invention reveals that proteins with the same primary sequence might have different biological activities. As illustrated in the following example, this invention discloses two proteins with identical amino acid sequences but with different activities. The efficacy of this activity may sometimes be improved and, sometimes, the protein with changed spatial configuration would reveal new function.

Also disclosed are equivalents or mimics of the recombinant interferon as described above.

An equivalent is a molecule which is similar in function to the compound interferon. An equivalent could be a deletion, substitution, or replacement mutant of the original sequence.

Mimics could be a peptide, polypeptide or a small chemical entity.

The interferon described herein includes but is not limited to interferon $\alpha$, $\beta$, or $\omega$. In an embodiment, it is IFN-1a, IFN-2b or other mutants.

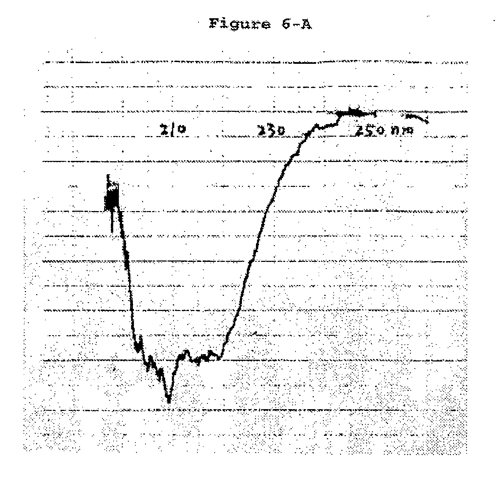

In an embodiment, the super-compound interferon disclosed has higher efficacy than the interferon described in U.S. Patent Nos. 4,695,623 or 4,897,471. This super-compound interferon is believed to have unique secondary or tertiary structure. (See e.g. Figure 6.)

The super-compound interferon described herein has spatial structure change(s) resulting from the changes of its production process.

The above-described super-compound interferon may be produced by a high-efficiency expression system which uses a special promoter. In an embodiment, the promoter is $P_{BAD}$. As could be easily appreciated by other ordinary skilled artisans. Other inducible promoters, such as heat shock promoters or heavy metal inducible promoters, may be used in this invention.

The super-compound interferon may also be produced with its gene as artificially synthesized cDNA with adjustment of its sequence from the wild-type according to codon preference of E. Coli. Extensive discussion of said codon usage (preference) may be found in U.S. Patent No. 4,695,623. See e.g. column 6, line 41 - column 7, line 35.

The above-described super-compound interferon possesses anti-viral or anti-tumor activity, and; therefore, is useful in inhibiting, preventing and treating viral diseases, tumors, or cancers.

As used herein, viral diseases include, but are not limited to, hepatitis A, hepatitis B, hepatitis C, other types of hepatitis, infections caused by Epstein-Barr virus, Cytomegalovirus, herpes simplex viruses, other herpes viruses, papovaviruses, poxviruses, picornaviruses, adenoviruses, rhinoviruses, human T-cell leukemia virus I, human T-cell leukemia virus II, or human T-cell leukemia virus III.

Viral upper respiratory infection, alternative names common cold, colds. This is a contagious viral infection of the upper respiratory tract characterized by inflammation of the mucous membranes, sneezing, and a sore throat. It is usually caused by over 200 different viruses, known as rhinoviruses. Colds are not caused by the same viruses responsible for influenza. Colds are spread through droplets from the coughing or sneezing of others with a cold or by hand contact with objects contaminated by someone with a cold. The incidence of colds is highest among children, and the incidence decreases with age because immunity to the virus causing the cold occurs after the illness. Gradually, immunity to a wide variety of viruses that cause colds is developed in adults. Children may have 10 colds a year, and adults may have 3 colds a year.

The U.S. Centers for Disease Control and Prevention have estimated that the average annual incidence of upper respiratory tract infections (URIs) in the United States is 429 million episodes, resulting in more than $2.5 billion in direct and indirect healthcare costs.

The common cold is most often caused by one of several hundred rhinoviruses (52%), but coronaviruses (8%) or the respiratory syncytial virus (7%) may also lead to infection. Other viruses, such as influenza (6%), parainfluenza, and adenoviruses, may produce respiratory symptoms, but these are often associated with pneumonia, fever, or chills.

Colds occur in a seasonal pattern that usually begins in mid-September and concludes in late April to early May. The common cold is quite contagious and can be transmitted by either person-to-person contact or airborne droplets. Upper respiratory symptoms usually begin 1 to 2 days after exposure and generally last 1 to 2 weeks, even though viral shedding and contagion can continue for 2 to 3 more weeks. Symptoms may persist with the occurrence of complications such as sinusitis or lower respiratory involvement such as bronchitis or pneumonia.

The common cold has a variety of overt symptoms, including malaise, nasal stuffiness, rhinorrhea, nonproductive cough, mild sore throat, and, in some cases, a low-grade fever. Because of the similarity of symptoms, a cold may be mistaken for perennial allergic rhinitis, but allergies can usually be ruled out because of the differences in chronicity.

If a patient presents with a viral URI, the spectrum of remedies is extensive. Since most of these infections are self-limiting, clinicians usually recommend rest and fluids, but other treatments include environmental and nutritional therapies, over-the-counter and prescription decongestant and antihistamine products, new antihistamine and anticholinergic nasal formulations, and antibiotics. Table 1 lists commonly used cough and cold medications and their side effects.

**Table 1. A Profile of Common Cough and Cold Medications and their side effects**

| Medication | Purpose | Side Effects and Special Considerations |
|---|---|---|
| Aerosolized beta2 agonists (eg, albuterol) | Reverse postinflammatory bronchospasm | Raises heart rate and may cause tremor |
| Alcohol-based liquid combination products | Treat multiple symptoms | Potential drowsiness and coordination problems |
| Alphal agonists (oral) (eg, pseudoephedrine, phenylpropanolamine) | Decongestion | May cause tachycardia, nervousness, transient stimulation, dizziness, drowsiness, elevation of blood pressure |
| Anticholinergic compounds: Ipratropium bromide (topical) | Drying | May cause nasal dryness and occasional epistaxis |
| Other anticholinergics (eg, methscopolamine, atropine, hyoscyamine) | Drying | May cause orthostasis, dysfunction of heat regulation, dry mouth, constipation |
| Antihistamines (oral) (eg, chlorpheniramine, diphenhydramine) | Drying | Drowsiness, dry mouth, orthostatic hypertension |
| Benzonatate capsules | Cough suppression, local anesthesia | Chewing can numb the mouth; can cause sedation, dizziness |
| Codeine, hydrocodone | Cough suppression | Drowsiness, constipation, nausea |
| Dextromethorphan | Cough suppression | Drowsiness possible, but side effects uncommon |
| Guaifenesin | Promote expectoration (mucolysis) | No side effects; must be taken with lots of water to improve efficacy |
| Topical decongestants (eg, oxymetazoline, phenylephrine) | Decongestion | Local burning; prolonged use may cause dependence |
| Zinc and vitamin C lozenges | Possible reduction in symptom severity and duration | Possible taste disturbance, increase of oxalate stones if susceptible |
| Abstract from http://www.physsportsmed.com/issues/1998/02feb/swain.htm | | |

### The Usage of Super-compound Interferon to Prevent or Treat URI

Nearly 70~80% URI are caused by viruses such as respiratory Syncytical virus, adenovirus, rhinovirous, coxsackie virus, corona virus and its variant, influenza A virus and its variant, influenza B virus and its variant, parainfluenza virus and its variant, or enterovirus and its variant. A main cause of URI in adults is from rhinovirous. For children, respiratory syncytical virus and parainfluenza virus are two leading causes of URI.

Super-compound interferon plays an important role in the fight against virus that causes URI. Super-compound interferon gains its anti-virus affects mainly via two mechanisms:

1. Attach to surface of sensitive cells and induce them to produce anti-virus protein, then block the duplication and reproduction of viruses in vivo.
2. Super-compound interferon can adjust immune response, including T-cell immune response, activity of NK cell, the phagocytosis function of monokaryon, and even formation of some antibodies in vivo.

In treatment for URI, Super-compound interferon can be directly applied to the affected area via a spray inspiration. This method of treatment allows the interferon to reach the target cells first hand. Consequently, marketing the supply as a spray, rather than via oral or injection, would be safer and more effective for administrating the interferon.

## Usage of Super-compound Interferon to Prevent or Treat SARS

With the consent of the *Sichuan working group on SARS prevention and control*, the distribution of Super-compound interferon began in May of 2003. Super-compound interferon spray was allocated to doctors and nurses in hospitals, populated areas with a high risk for SARS, and to the *National research group on prevention and control of SARS*. Among the 3,000 users as of December 19, 2003, there were no reports of any side effects connected to the use of the spray. Furthermore, none of the doctors and nurses, the people of Sichuan Province, or other organizations that have used the Super-compound interferon spray has been infected by SARS.

Therefore, this invention provides a method for inhibiting, preventing or treating virus replication or virus-infected cells by contacting said virus or infected cells with an effective amount of the super-compound interferon **as defined above**.

This super-compound interferon is useful in inhibiting, preventing or treating the following cancers or tumors:

| | | |
|---|---|---|
| Cancer | Skin Cancer | Basal Cell Carcinoma |
| | | Malignant Melanoma |
| | Renal cell carcinoma | |
| | Liver Cancer | |
| | Thyroid Cancer | |
| | Rhinopharyngeal Cancer | |
| | Solid Carcinoma | Prostate Cancer |
| | | Stomach/Abdominal Cancer |
| | | Esophageal Cancer |
| | | Rectal Cancer |
| | | Pancreatic Cancer |
| | | Breast Cancer |
| | Ovarian Cancer & Superficial Bladder Cancer | |
| | Hemangioma | |
| | Epidermoid Carcinoma | Cervical Cancer |
| | | Non-small Cell Lung Cancer |
| | | Small Cell Lung Cancer |
| | | Glioma |
| Malignant Hemal Disease | Leucocythemia | Acute Leucocythemia |
| | | Chronic Leucocythemia |
| | Chronic Myelocytic Leukemia | |
| | Hairy Cell Leukemia | |
| | Lymphadenoma | |
| | Multiple Myeloma | |
| | Polycythemia Vera | |
| Others | Kaposi's Sarcoma | |

**Patient #1.** A female patient with ovarian cancer started receiving injections. She received 15$\mu$g injections on July 14th, July 16th, July 18th, July 20th, and July 22nd. On July 14th, 2000ml of peritoneal fluid was observed. The patient underwent chemotherapy on July 22nd. On August 3rd, the patient's peritoneum was opened. 2l of fluid was expected to be found, but only 200ml of fluid was observed. The left and right ovaries and lymphatic nodes were cancerous. All other organs were clear.

**Patient #2.** A kidney cancer patient was treated in the following manner. In a half-month period, the patient was given 3 injections of 9μg of rSIFN-co and 3 injections of 15μg of rSIFN-co. In the one full month following these injections, he received 9μg and 15μg injections of rSIFN-co every other day. A kidney biopsy showed no metastasis after this course of treatment. The patient showed a full recovery. Every half year after recovery, the patient received 15μg injections of rSIFN-co 15 times over a one-month period.

Accordingly, this invention provides a method for inhibiting tumor or cancer cell growth by contacting the super-compound interferon as defined above with said tumor or cancer cells.

In a further embodiment, the super-compound interferon inhibits the DNA duplication and secretion of HBsAg and HBeAg of Hepatitis B Virus.

This invention also provides artificial gene codes for the super-compound interferon. It is within the ordinary skill to design an artificial gene. Many methods for generating nucleotide sequence and other molecular biology techniques have been described previously. See for example, Joseph Sambrook and David W. Russell, Molecular Cloning: A laboratory Manual, December 2000, published by Cold Spring Harbor Laboratory Press.

This invention provides a vector comprising the polynucleotide sequence shown in Fig. 1 which codes for the super-compound interferon as defined above.

This invention provides an expression system comprising the vector as defined above. The cells include, but are not limited to, prokaryotic or eukaryotic cells.

This invention also provides a host cell comprising the vector as defined above.

This invention provides a process for production of recombinant super-compound interferon as defined above comprising introducing the polynucleotide sequence shown in Fig. 1 with selected codon preference into E. Coli host, culturing said introduced host in an appropriate condition for the expression of said compound interferon and harvesting the expressed compound interferon.

The process may comprise extraction of super-compound interferon from fermentation broth, collection of inclusion body, denaturation and renaturation of the harvested protein.

The process may maintain the high efficacy even when the super-compound interferon is used with an agent and in a particular concentration. The process also comprises separation and purification of the super-compound interferon. The process further comprises lyophilization of the purified super-compound interferon. The process comprises production of liquid injection of super-compound interferon.

This invention also provides the produced super-compound interferon by the above processes.

This invention provides a composition comprising the recombinant super-compound interferon **as defined above** and a suitable carrier.

This invention provides a pharmaceutical composition comprising the recombinant super-compound interferon **as defined above** and a pharmaceutically acceptable carrier.

This invention provides a method for treating or preventing viral diseases or tumors in a subject comprising administering to the subject an effective amount of the super-compound interferon **as defined above**.

This invention provides the above-described method wherein the viral diseases include, but are not limited to, hepatitis A, hepatitis B, hepatitis C, other types of hepatitis, infections of viruses caused by Epstein-Barr virus, Cytomegalovirus, herpes simplex viruses, or other type of herpes viruses, papovaviruses, poxviruses, picornaviruses, adenoviruses, rhinoviruses, human T-cell leukemia viruses I, or human T-cell leukemia viruses II, or human T-cell leukemia virus III.

This invention provides the above-described method wherein super-compound interferon was administered via orally via vein injection, muscle injection, peritoneal injection, subcutaneous injection, nasal or mucosal administration, or by inhalation via an inspirator.

This invention provides the above-described method wherein super-compound interferon was administered following the protocol of injections of 9μg or 15μg every two days, 3 times a week, for 24 weeks.

It was surprising to find that rSIFN-co, the spatial structure of which has been changed, is not only a preparation to inhibit the DNA duplication of hepatitis B, but to inhibit the secretion of HBsAg and HBeAg on 2.2.15 cells.

One objective of this invention is to offer a preparation of rSIFN-co to directly inhibit the DNA duplication of hepatitis B viruses and the secretion of HBeAg and HBsAg of hepatitis B and decrease them to normal levels.

In one embodiment, rSIFN-co was produced with recombinant techniques. On the condition of fixed amino acid sequence, the IFN DNA was redesigned according to the *E. Coli.* codon usage and then the rSIFN-co gene was artificially synthesized. rSIFN-co cDNA was cloned into the high-expression vector of *E. Coli.* by DNA recombinant techniques, and a high expression of rSIFN-co was gained by using of induce/activate-mechanism of L-arabinose to activate the transcription of $P_{BAD}$ promoter.

Compared with usual thermo-induction, pH induction and IPTG induction systems of genetic engineering, arabinose induction/activation system has some advantages: (1) Common systems relieve promoter function by creating a "derepression" pattern. Promoters then induce downstream gene expression. Temperature and pH change and the addition of IPTG cannot activate promoters directly.

In the system disclosed herein, L-arabinose not only deactivates and represses but also activates the transcription of $P_{BAD}$ promoter which induces a high expression of rSIFN-co. Therefore, the arabinose induction/activation system is a more effective expression system. (2) The relationship between Exogenous and L-arabinose dosage is linear. This means the concentration of arabinose can be changed to adjust the expression level of the exogenous gene. Therefore, it is easier to control the exogenous gene expression level in *E. Coli.* by arabinose than by changing temperature and pH value. This characteristic is significant for the formation of inclusion bodies. (3) L-arabinose is resourceful, cheap and safe, which, on the contrary, are the disadvantages of other inducers such as IPTG. This embodiment creates an effective and resistant rSIFN-co-expressing *E. Coli.* engineering strain with an L-arabinose induction/activation system.

The strain is cultivated and fermented under suitable conditions to harvest the bacterial bodies. Inclusion bodies are then purified after destroying bacteria and washing repeatedly. The end result, mass of high-purity, spatial-configuration-changed rSIFN-co protein for this invention and for clinical treatment, was gained from denaturation and renaturation of inclusion bodies and a series of purification steps.

The following are some rSIFN-co preparations: tablets, capsules, liquids for oral consumption, pastes, injections, sprays, suppositories, and solutions. Injections are recommended. It is common to subcutaneously inject or veininject the medicine. The medicine carrier could be any acceptable medicine carrier, including carbohydrates, cellulosum, adhesive, collapse, emollient, filling, add-dissolving agent, amortization, preservative, thickening agent, matching, etc. This invention also provides a pharmaceutical composition comprising the above composition and a pharmaceutically acceptable carrier.

For the purposes of this invention, "pharmaceutically acceptable carriers" means any of the standard pharmaceutical carriers. Examples of suitable carriers are well known in the art and may include, but are not limited to, any of the standard pharmaceutical carriers, such as a phosphate buffered saline solution and various wetting agents. Other carriers may include additives used in tablets, granules, capsules, etc. Typically such carriers contain excipients such as starch, milk, sugar, certain types of clay, gelatin, stearic acid or salts thereof, magnesium or calcium stearate, talc, vegetable fats or oils, gum, glycols or other known excipients. Such carriers may also include flavor and color additives or other ingredients. Compositions comprising such carriers are formulated by well-known conventional methods.

This invention provides a method for preventing or treating Severe Acute Respiratory Syndrome, or virus-induced upper respiratory diseases, of a subject comprising administering to the subject an effective amount of recombinant super-compound interferon **as defined above**.

In an embodiment of the above method, the interferon is $\alpha$, $\beta$, or $\omega$.

The super-compound interferon **as defined above** may be administered orally, via vein injection, muscle injection, peritoneal injection, subcutaneous injection, nasal or mucosal administration, or by inhalation via an inspirator.

In an embodiment, the interferon is delivered by a spray device.

In a specific embodiment, the device is described in Figure 7.

In one of the embodiments, the interferon is lyophilized. This invention provides a method for inhibiting the causative agent of Severe Acute Respiratory Syndrome, or virus-induced upper respiratory diseases, comprising contacting the agent with an effective amount of super-compound interferon **as defined above**.

It is determined that the causative agent of SARS is a virus. <u>See</u> eg. Rota et al (2003), Characterization of a Novel Coronavirus Associated with Severe Acute Respiratory Syndrome. Science 1085952 www.sciencexpress.org and Marra, et al. (2003), The Genome Sequence of the SARS-Associated Coronavirus. Science 1085853 www.sciencexpress.org. This invention also provides a method for inhibiting Severe Acute Respiratory Syndrome virus or Severe Acute Respiratory Syndrome virus-infected cells, or virus-induced upper respiratory diseases, or cells infected with viruses capable of inducing upper respiratory diseases, comprising contacting an effective amount of the super-compound interferon **as defined above** with said virus or cell. This contact could be direct or indirect.

This invention provides a composition comprising an effective amount of the super-compound interferon **as defined above** capable of inhibiting Severe Acute Respiratory Syndrome virus or Severe Acute Respiratory Syndrome virus-infected cells, or virus-induced upper respiratory diseases, or cells infected with viruses capable of inducing upper respiratory diseases, and a suitable carrier.

This invention provides a composition comprising an effective amount of the super-compound interferon **as defined above** capable of preventing or treating Severe Acute Respiratory

Syndrome, or virus-induced upper respiratory diseases, of a subject and a suitable carrier. This invention provides a pharmaceutical composition comprising an effective amount of the recombinant super-compound interferon **as defined above** capable of inhibiting Severe Acute Respiratory Syndrome virus or Severe Acute Respiratory Syndrome virus-infected cells, or virus-induced upper respiratory diseases, and a pharmaceutically acceptable carrier.

This invention provides a pharmaceutical composition comprising an effective amount of the recombinant super-compound interferon **as defined above** capable of preventing or treating Severe Acute Respiratory Syndrome, or virus-induced upper respiratory diseases, in a subject and a pharmaceutically acceptable carrier.

This invention provides a device to deliver the above-described pharmaceutical composition. In a preferred embodiment, the subject is a human. As it can easily be appreciated, the super-compound interferon **as defined above** can be used in other animals or mammals.

This invention provides a method for preventing Severe Acute Respiratory Syndrome or virus-induced upper respiratory diseases, in humans comprising application of the super-compound interferon **as defined above** three times a day via a spray which contains twenty micrograms of interferon, equal to ten million units of activity in three milliliter. This invention will be better understood from the examples which follow. However, one skilled in the art will readily appreciate that the specific methods and results discussed are merely illustrative of the invention as described more fully in the claims which follow thereafter.

## EXPERIMENTAL DETAILS

### EXAMPLE 1

rSIFN-co is a new interferon molecule constructed according to conservative amino acids in human IFN-$\alpha$ subtype using genetic engineering methods. It has been proven that rSIFN-co has broad-spectrum IFN activity, such as high antivirus and tumor inhibition activity, especially for effectively treating hepatitis C.

E. Coli. codon was used to redesign rSIFN-co cDNA and then artificially synthesize cDNA of rSIFN-co from published rSIFN-co DNA sequences and deduced amino acid sequences (Figure 1).

In order to get pure rSIFN-co protein, rSIFN-co cDNA was cloned into E. Coli. high-expression vector, and L-arabinose, which can activate strong P$_{BAD}$ promoter in vectors, was used to induce high expression of rSIFN-co gene.

### Synthesis of *E. Coli.* cDNA Sequence

### Redesign of rSIFN-co cDNA sequence

rSIFN-co cDNA was redesigned according to the codon usage of *E. Coli.* to achieve high expression in *E. Coli.* Deduced amino acid sequence from the redesigned cDNA sequence of rSIFN-co is completely coincidental with primitive amino acid sequence of published rSIFN-co (Figure 1).

### rSIFN-co cDNA sequence synthesis

### rSIFN-co cDNA 5'-terminus and 3'- terminus semi-molecular synthesis

Two semi-moleculars can be directly synthesized: rSIFN-co cDNA 5'- terminus 280bp (fragment I) and 3'-terminus 268bp(fragment II) by PCR. There are 41bp overlapping among fragment II and fragment I.

(1) Chemical synthesis oligodeoxynucleotide fragment:

Oligomer A: SEQ ID No. 5

5'ATGTGCGACCTGCCGCAGACCCACTCCCTGGGTAACCGTCGTGCTCTGATCCTGCTGGCTCA
GATGCGTCGTATCTCCCCGTTCTCCTGCCTGAAAGACCGTCACGAC3'

Oligomer B: SEQ ID No. 7

5'CTGAAAGACCGTCACGACTTCGGTTTCCCGCAGGAGAGGTTCGACGGTAACCAGTTCCAGA
AGCTCAGGCTATCTCCGTTCTGCACGAAATGATCCAGCAGACCTTC3'

Oligomer C: SEQ ID No. 8

5'GCTGCTGGTACAGTTCGGTGTAGAATTTTTCCAGCAGGGATTCGTCCCAAGCAGCGGAGGAG
TCTTTGGTGGAGAACAGGTTGAAGGTCTGCTGGATCATTTC3'

Oligomer D: SEQ ID No. 9

5'ATCCCTGCTGGAAAAATTCTACACCGAACTGTACCAGCAGCTGAACGACCTGGAAGCTTGCG
TTATCCAGGAAGTTGGTGTTGAAGAAACCCCGCTGATGAAC3'

Oligomer E: SEQ ID No. 10

5'GAAGAAACCCCGCTGATGAACGTTGACTCCATCCTGGCTGTTAAAAAATACTTCCAGCGTAT
CACCCTGTACCTGACCGAAAAAAAATACTCCCCGTGCGCTTGGG3'

Oligomer F: SEQ ID No. 11

5'TTATTCTTTACGACGCAGACGTTCCTGCAGGTTGGTGGACAGGGAGAAGGAACGCATGATTT
CAGCACGAACAACTTCCCAAGCGCACGGGGAGTATTTTTTTTCGGTCAGG3'

PCR I for Fragment I: oligodeoxynucleotide B as template, oligodeoxynucleotide A and C as primers, synthesized 280 bp Fragment I.
PCR I mixture (units: μl)

| | |
|---|---|
| sterilized distilled water | 39 |
| 10×Pfu buffer ( Stratagen American Ltd. ) | 5 |
| dNTP mixture( dNTP concentration 2.5 mmol/L ) | 2 |
| Oligomer A primer ( 25 μmol/L ) | 1 |
| Oligomer C primer ( 25 μmol/L ) | 1 |
| Oligomer B template( 1 μmol/L ) | 1 |
| Pfu DNA polymerase( stratagem American Ltd. ) (25 U/μl ) | 1 |
| Total volume | 50μl |
| PCR cycle: 95 I | |

PCR II for Fragment II: oligodeoxynucleotide E as template, oligodeoxynucleotide D and F as primers, synthesized 268bp Fragment II.

| PCR II mixture | (units: μl) |
|---|---|
| sterilized distilled water | 39 |
| 10×Pfu buffer( Stratagen American Ltd. ) | 5 |
| dNTP mixture( dNTP concentration 2.5mmol/L ) | 2 |
| Oligomer D primer( 25 μmol/L ) | 1 |
| Oligomer F primer( 25 μmol/L ) | 1 |
| Oligomer E template ( μmol/L) | 1 |
| Pfu DNA polymerase ( Stratagen American Ltd.. ) ( 25U/μl ) | 1 |
| Total volume | 50μl |
| PCR cycle: the same as PCR I | |

**Assembling of rSIFN-co cDNA**

Fragment I and II were assembled together to get the complete cDNA molecular sequence of rSIFN-co using the overlapping and extending PCR method. Restriction enzyme Nde I and Pst I were introduced to clone rSIFN-co cDNA sequence into plasmid.

(1) Chemical synthesis primers

Oligomer G: 5'ATCGGCCATATGTGCGACCTGCCGCAGACCC3' SEQ ID No. 12

Oligomer H: 5'ACTGCCAGGCTGCAGTTATTCTTTACGACGCAGACGTTCC3' SEQ ID No. 13

(2) Overlapping and extending PCR

| PCR mixture | (units: μl) |
|---|---|
| sterilized distilled water | 38 |
| 10×Pfu buffer( Stratagen American Ltd. ) | 5 |
| dNTP mixture(dNTP concentration 2.5mmol/L) | 2 |
| primer G ( 25 μmol/L ) | 1 |
| primer H ( 25 μmol/L ) | 1 |
| *fragment I preduction (1 μmol/L) | 1 |
| *fragment II preduction (1 μmol/L) | 1 |
| Pfu DNA polymerase ( Stratagen American Ltd. ) ( 2.5U/μl ) | 1 |
| Total volume | 50μ |

*Separate and purify PCR production with StrataPrep PCR purification kit produced by Stratagen American Ltd. And dissolve into sterilized distilled water.
PCR cycle: the same as PCR I

**rSIFN-co gene clone and sequence analysis**

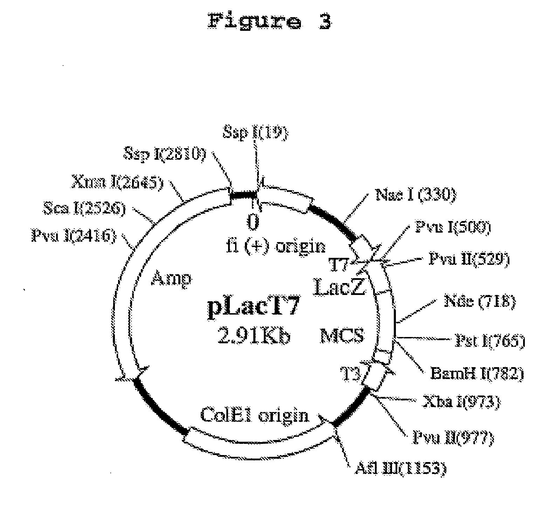

pLac T7 plasmid as cloning vector. pLac T7 plasmid is reconstructed with pBluescript II KS(+) plasmid produced by Stratagen (Figure 3).

Purified PCR production of rSIFN-co cDNA with StrataPrep PCR purification kit. Digest cDNA and pLac T7 plasmid with NdeI and PstI. Run 1% agarose gel electrophoresis and separate these double-digested DNA fragments. Recover 507bp long rSIFN-co DNA fragment and 2.9kb plasmid DNA fragment. Ligate these fragments by T4 DNA ligase to form a recombinant plasmid. Transform $DH_{5\alpha}$ competent cells (Gibco) with the recombinant plasmid, culture at 37°C overnight. Identify the positive recombinant colony, named pHY-1.

Run DNA sequencing with SequiTherm™ Cycle Sequencing Kit produced by American Epicentre Technologies Ltd using L1-COR Model 4000L. Primers are T7 and T3 common sequence primer, the DNA sequencing result matches theoretic design.

Purify the rSIFN-co, sequence the N-terminus amino acids, the N-terminus amino acid sequence matches experimental design which is as follows:

```
N- Cys-Asp-Leu-Pro-Gln-Thr-His-Ser-Leu-Gly-Asn-Arg-Arg-Ala-
Leu- (SEQ ID No. 14)
```

**Construction, transformation, identification, and hereditary stability of expression vector**

**Construction and transformation of expression vector**

Digested *E. Coli.* expression vector pHY-4(see Figure 3) with Nde I to linearize and subsequently digest with Xba I. Run 1% agarose gel electrophoresis, and purify the 4.8kb pHY-4 Nde I -Xba I digest fragment with QIAEX II kit produced by QIAGEN Germany Ltd.

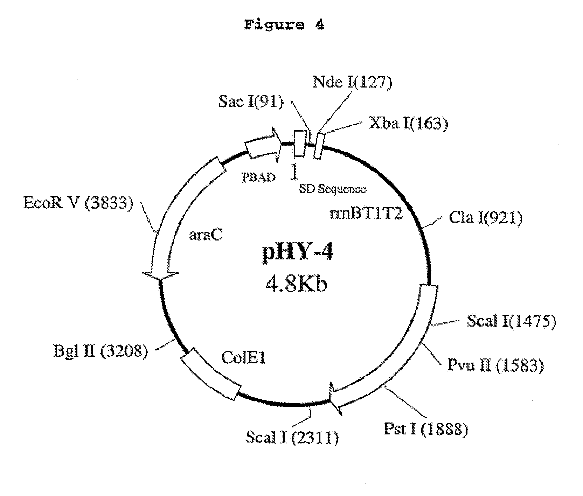

At the same time, the pHY-4 plasmid is double digested with Nde I-Xba I. Run 1% agarose gel electrophoresis and purify the 715bp fragment. Ligate the rSIFN-co and pHY-4 fragments with T4 DNA ligase to construct the recombinant plasmid (See Figure 4). Transform $DH_{5\alpha}$ competent cells with the recombinant plasmid. Spread the transformed cells on LB plate with Amp, 37°C culture overnight.

**Positive cloning strain screening**

Randomly choose *E. Coli.* colonies from above LB-plate, screening the positive strains containing recombinant vector by endonuclease digesting and PCR analysis. Name one of the positive recombinant plasmid pHY-5, and name the strain containing pHY-5 plasmid PVIII. Amplify and store the positive strain with glycerol in -80°C.

**High expression of rSIFN-co gene in E. *Coli.***

In pHY-5 plasmid, rSIFN-co gene is under the control of strong promoter $P_{BAD}$. This promoter is positively and negatively regulated by the product of the gene araC. AraC is a transcriptional regulator that forms a complex with arabinose. In the absence of arabinose, the AraC dimer binds $O_2$ and $I_1$, forming a 210bp loop. This conformation leads to a complete inhibition of transcription. In the presence of arabinose, the dimer is released from $O_2$ and binds $I_1$ and $I_2$ leading to transcription. Arabinose binding deactivates, represses, and even activates the transcription of $P_{BAD}$ promoter, which stimulates $P_{BAD}$, inducing high expression of rSIFN-co. rSIFN-co expression level in PVIII is more than 50% of the total *E. Coli.* protein.

**Summary**

RSIFN-CO is a new interferon molecule artificially built according to the conservative amino acid of human $\alpha$ interferons. It has been proven as an effective anti-hepatitis drug. In order to get enough pure rSIFN-co protein, a stable recombinant *E. Coli.* strain which highly expresses rSIFN-co protein was constructed.

First, according to published rSIFN-co amino acid sequence, *E. Coli.* codon was used to synthesize the whole cDNA of rSIFN-co. This DNA fragment was sequenced, proving that the 501bp codon sequence and TAA termination codon sequence are valid and identical to theocratic design. Subsequent analysis revealed that the N-terminus amino acid sequence and amino acid composed of rSIFN-co produced by the recombinant strain were both identical to the prediction.

The rSIFN-co cDNA was cloned into *E. Coli.* high-expression vector pHY-4 plasmid to construct the recombinant plasmid pHY-5. *E. Coli.* LMG194 strain was further transformed with pHY-4 plasmid to get stable rSIFN-co high-expression transformant. This transformant was cultured for 30 generations. The heredity of pHY-5 recombinant plasmid in *E. Coli.* LMG194 was normal and stable, and the expression of rSIFN-co was high and steady.

*E. Coli.* LMG194, which contains recombinant pHY-5 plasmid, is actually an ideal high-expression engineering strain.

**References**

1. Blatt LM, Davis JM, Klein SB. et al. The biologic activity and molecular characterization of a novel synthetic interferon-alpha species, consensus interferon. Journal of Interferon and Cytokine Research, 1996; 16 (7) :489-499.

2. Alton,K. et al: Production characterization and biological effects of recombinant DNA derived human IFN-$\alpha$ and IFN-$\gamma$ analogs. In: De Maeger E, Schellekens H. eds. The Biology of Interferon System.2nd ed. Amsterdam: Elsevier Science Publishers, 1983: 119-128

3. Pfeffer LM. Biologic activity of natural and synthetic type 1 interferons. Seminars in Oncology, 1997;24 (3 suppl 9):S9-63--S9-69.

4. Ozes ON, Reiter Z, Klein S, et al. A comparison of interferon-con1 with natural recombinant interferons-(: antiviral, antiproliferative, and natural killer-inducing activities. J. Interferon Res., 1992; 12:55-59.

5. Heathcote EJL, Keeffe EB, Lee SS, et al. Re-treatment of chronic hepatitis C with consensus interferon. Hepatology, 1998;27(4):1136-1143.

6. Klein ML, Bartley TD, Lai PH, et al. Structural characterization of recombinant consensus interferon-alpha. Journal of Chromatography, 1988; 454:205-215.

7. The Wisconsin Package, by Genetics Computer Group, Inc. Copyright 1992, Medison, Wisconsin, USA

8. Nishimura, A et al: A rapid and highly efficient method for preparation of competent E. coli cells. Nuclei. Acids Res. 1990, 18:6169

9. All molecular cloning techniques used are from:Sambrook, J., E. F. Fritsch and T. Maniatis. Molecular Cloning:A laboratory manual,2nd ed. CSH Laboratory Press, Cold Spring Harbour, NY.1989.

10. Guzman, L. M et al: Tight regulation, modulation, and high-level express-ion by vectors containing the arabinose PBAD promoter. J. Bacteriol. 1995, 177:4121~ 4130.

rSIFN-co cDNA SEQUENCE DESIGNED ACCORDING TO E. COLI. CODON USAGE AND DEDUCED rSIFN-co AMINO ACID SEQUENCE

```
     5'      11      21      31      41      51
+1  M  C  D  L  P  Q  T  H  S  L   G  N  R  R  A  L  I  L  L  A
    1 ATGTGCGACC TGCCGCAGAC CCACTCCCTG GGTAACCGTC GTGCTCTGAT CCTGCTGGCT
      TACACGCTGG ACGGCGTCTG GGTGAGGGAC CCATTGGCAG CACGAGACTA GGACGACCGA
```

```
     5'      71      81      91     101     111
+1  Q  M  R  R  I  S  P  F  S  C   L  K  D  R  H  D  F  G  F  P
   61 CAGATGCGTC GTATCTCCCC GTTCTCCTGC CTGAAAGACC GTCACGACTT CGGTTTCCCG
      GTCTACGCAG CATAGAGGGG CAAGAGGACG GACTTTCTGG CAGTGCTGAA GCCAAAGGGC
```

```
     5'     131     141     151     161     171
+1   Q  E  E  F  D  G  N  Q  F  Q  K  A  Q  A  I  S  V  L  H  E
  121 CAGGAAGAAT TCGACGGTAA CCAGTTCCAG AAAGCTCAGG CTATCTCCGT TCTGCACGAA
      GTCCTTCTTA AGCTGCCATT GGTCAAGGTC TTTCGAGTCC GATAGAGGCA AGACGTGCTT
```

```
     5'     191     201     211     221     231
+1  M  I  Q  Q  T  F  N  L  F  S  T  K  D  S  S  A  A  W  D  E
```

181 ATGATCCAGC AGACCTTCAA CCTGTTCTCC ACCAAAGACT CCTCCGCTGC TTGGGACGAA
    TACTAGGTCG TCTGGAAGTT GGACAAGAGG TGGTTTCTGA GGAGGCGACG AACCCTGCTT

    5'     251    261    271    281    291

+1 S L L E K F Y T E L Y Q Q L N D L E A C

241 TCCCTGCTGG AAAAATTCTA CACCGAACTG TACCAGCAGC TGAACGACCT GGAAGCTTGC
    AGGGACGACC TTTTTAAGAT GTGGCTTGAC ATGGTCGTCG ACTTGCTGGA CCTTCGAACG

    5'       311     321     331     341     351

+1  V  I  Q   E  V  G  V   E  E  T  P  L  M  N   V  D  S  I  L  A

301 GTTATCCAGG AAGTTGGTGT TGAAGAAACC CCGCTGATGA ACGTTGACTC CATCCTGGCT
    CAATAGGTCC TTCAACCACA ACTTCTTTGG GGCGACTACT TGCAACTGAG GTAGGACCGA

    5'       371     381     391     401     411

+1  V  K  K  Y  F  Q  R  I  T  L  Y  L  T  E  K  K  Y  S  P
C

361 GTTAAAAAAT ACTTCCAGCG TATCACCCTG TACCTGACCG AAAAAAAATA CTCCCCGTGC
    CAATTTTTTA TGAAGGTCGC ATAGTGGGAC ATGGACTGGC TTTTTTTTAT GAGGGGCACG

    5'       431     441     451     461     471

+1  A  W  E  V  V  R  A  E  I  M  R  S  F  S  L  S  T  N  L
Q

421 GCTTGGGAAG TTGTTCGTGC TGAAATCATG CGTTCCTTCT CCCTGTCCAC CAACCTGCAG
    CGAACCCTTC AACAAGCACG ACTTTAGTAC GCAAGGAAGA GGGACAGGTG GTTGGACGTC

    5'       491     501

+1   E   R   L   R   R   K   E   #

481 GAACGTCTGC GTCGTAAAGA ATAA
    CTTGCAGACG CAGCATTTCT TATT

Nucleotide sequence is SEQ ID No. 1 and amino acid sequence is SEQ ID No. 2

**EXAMPLE 2**

**Separation and purification of rSIFN-co**

1. Fermentation

Inoculate the recombinant strain in LB media, shaking (200 rpm) under 37°C overnight (approximate 18 h), then add 30% glycerol to the fermentation broth to get final concentration of 15%, allotted to 1 ml tube and kept in - 20°C as seed for production.

Add 1% of the seed to LB media, shaking (200 rpm) under 37°C overnight to enlarge the scale of the seed, then add to RM media with a ratio of 10%, culturing under 37°C. Add arabinose (20% solution) to 0.02% as an inductor when the OD600 reaches about 2.0. 4 hours after that, stop the culture process, collect the bacteria by centrifuge, resuspend the pellet with buffer A, and keep in -20°C overnight. Thaw and break the bacteria by homogenizer, then centrifuge. Wash the pellet with buffer B, buffer C, and distilled water to get a relatively pure inclusion body.

## 2. Denaturation and renaturation

Dissolve the inclusion body in Guanidine-HCl (or urea) of 6 mol/L. The solution will be a little cloudy. Centrifuge it at a speed of 10000 rpm. Determine the protein concentration of the supernatant. This supernatant is called "denaturation solution." Add the denaturation solution to renaturation buffer, and keep the final protein concentration under 0.3 mg/ml. It is better to add the totally denatured solution in three steps instead of one step. Keep the solution overnight under 4°C. Afterwards, dialyze 10 mol/L,5 mol/L PB buffer and distilled water, then adjust its pH by 2 mol/L HAc-NaAc. Let it stand, then filtrate.

## 3. Purification

POROS HS/M anion exchange chromatography:

Equivalent column with 20 mmol/L HAc-NaAc(pH 5.0)
↓
Load samples at a speed of 30 ml/min
↓
Wash with 20 CV 20 mmol/L HAc-NaAc(pH 5.0)
↓
5 CV of 0.15 mol/L NaCl+20 mmol/L HAc-NaAc(pH 5.0)wash
↓
3 CV of 0.18 mol/L NaCl+20 mmol/L HAc-NaAc(pH 5.0)wash
↓
0.25 mol/L NaCl + 20 mmol/L HAc-NaAc(pH 5.0)elute target protein

Chelating sepharose™ fast flow: Add PB buffer of 0.2 mol/L(pH 6.6)and NaCl of 4 mol/L in the solution from HS to adjust solution pH to pH 6.0 and NaCl concentration to 1 mol/L.

Column with buffer D
↓
Loading at a rate of 1 ml/min
↓
Wash with buffer E
↓
Wash with buffer F
↓
Elute with buffer G

Condense the eluted solution by POROS HS/M. Sometimes a purification by sephacryl S-100 step can be added to meet stricter purity requirements.
Note:

Buffer A: 100 mmol/L Tris-HCl,pH 7.5-10 mmol/L EDTA-100 mmol/L NaCl
Buffer B: 50 mmol/L Tris-HCl,pH 7.5-1 mol/L Urea-10 mmol/L EDTA-0.5% Triton X-100
Buffer C: 50 mmol/L Tris-HCl,pH 7.5-2 mol/L Urea-10 mmol/L EDTA-0.5% Triton X-100
Buffer D: 1 mol/L NaCl ---50 mmol/L $Na_2HPO_4$ (pH 5.5)
Buffer E: 1 mol/L NaCl ---50 mmol/L $Na_2HPO_4$ (pH 5.0)

Buffer F: 1 mol/L NaCl ---50 mmol/L $Na_2HPO_4$ (pH 4.0)
Buffer G: 1 mol/L NaCl ---50 mmol/L $Na_2HPO_4$ (pH 3.6)

Renaturation buffer : 0.5 mol/L Arginine-150 mmol/L Tris-HCl, pH 7.5-0.2 mmol/L EDTA

| | |
|---|---|
| **LB Media:** | 1 L |
| Tryptone | 10 g |
| Yeast extracts | 5 g |
| NaCl | 10 g |
| **RM Media:** | 1 L |
| Casein | 20 g |
| MgCl | 1 mmol/L (0.203 g) |
| $Na_2HPO_4$ | 4 g; |
| $KH_2PO_4$ | 3 g, |
| NaCl | 0.5 g |
| $NH_4Cl$ | 1 g |

After purification, the buffer was changed to PBS (pH 7.0) along with the step of condensing by POROS HS/M. This is called the "Protein Stock Solution." It can be directly used in the preparation of injections or sprays, or stored at 2-8°C.

Formula for injection:

| | Solution | Lyophilized powder |
|---|---|---|
| Solution of rSIFN-co | 34.5 $\mu$g/ml | 34.5 $\mu$g/ml |
| PB (pH7.0) | 25mmol/L | 10mmol/L |
| Glycine | --- ---- -- | 0.4mol/L |
| NaCl | 0.1mol/L | ---- --- --- |

| For spray: | |
|---|---|
| EDTA | 0.01% |
| Tween 80 | 0.05% |
| Trisodium citrate | 10mmol/L |
| Glycerol | 1.26% |
| Sodium Chloride | 0.03% |
| Phenylmethanol | 0.5% |
| HSA | 0.1% |
| rSIFN-co | 10 $\mu$g/ml |

## QUALITY CONTROL PROCESS

During purification, tests for protein content, protein purity, specific activity and pyrogen are conducted after each step. When the stock solution is obtained, all the tests listed in the table are done one after the other.

The quality of the product is controlled according to "Chinese Requirements for Biologics."

1. Original protein solution

Lowry

| **Item of Test** | **Method** |
|---|---|
| **Protein Stock Solution:** | |
| Test for Protein Content | Lowry |

(continued)

| Item of Test | Method |
|---|---|
| **Protein Stock Solution:** | |
| Test for Protein Purity | Non-reductive SDS-PAGE (sodium dodecyl sulfate polyacrylamide gel electrophoresis ) HPLC Analysis |
| Test for Molecular Weights | Reductive SDS-PAGE |
| Test for Specific Activity | According to Method in "Specific Activity Test of Interferon |
| Test for Leftover Exogenetic DNA | Using DNA Labeling and Detection Kit |
| Test for Activity of Leftover Antibiotics | According to Method in "Chemical and Other Test Methods for Biologics" |
| Test for Bacterial Endotoxin | According to Method in "Requirements for Bacterial Endotoxin Test of Biologics" |
| Test for Isoelectronic Point | Isoelectric Focusing Electrophoresis |
| Test for Identify Characteristics of the Protein | UV spectrum (range of wavelength: 190-380nm) |
| | Peptide Mapping (hydrolyzed |
| | by pancreatic enzyme, analyzed by C-18 column) |
| | N-terminal Sequence Test |
| | C-terminal Sequence Test |
| | Circular Dichroism |
| | Amino Acid Analysis |
| | |
| **Semi-finished Product** | |
| Test for Bacterial Endotoxin | According to Method in "Requirements for Bacterial Endotoxin Test of Biologics" |
| | |
| **Product** | |
| Appearance Check | |
| Chemical | According to Method in "Chemical and Other Test Methods for Biologics" |
| Test for Specific Activity | According to Method in "Specific Activity Test of Interferon |
| Sterility Test | According to Method in "c" |
| Abnormal Toxicity Test | Test on Mouse |
| Pyrogen Test | According to Method in "Requirements for Pyrogen Test of Biologics" |
| Test for Stability of Product | |
| Note: "Chemical and Other Test Methods for Biologics", "Requirements for Pyrogen Test of Biologics" and "Requirements for Bacterial Endotoxin Test of Biologics" all can be found in the "Chinese Requirements for Biologics." "Chinese Requirements for Biologics," PAN Zhengan, ZHANG Xinhui, DUAN Zhibing, et al. Chinese Biologics Standardization committee. Published by Chemical Industry Publishing Company, 2000. | |

## EXAMPLE 3

### Stability of lyophilized Powder of Recombinant Super-Compound Interferon Injection

The stability experiments were carried out with samples of lyophilized powder of recombinant super-compound interferon (rSIFN-co) injection in two specifications and three batches. The experiments started in April 2000.

## 1. Sample Source

Samples were supplied by Sichuan Huiyang Life-engineering Ltd., Sichuan Province. Lot: 990101-03, 990101-05, 990102-03, 990102-05, 990103-03, 990103-05

## 2. Sample Specifications

Every sample in this experiment should conform with the requirements in the table below.

Table 1 Standard of Samples in Experiment

| Items | Standards |
|---|---|
| 1. Appearance | white loose powder |
| 2. Dissolving time | dissolve rapidly in injection water( within 2 min) at room temperature |
| 3. Clarity | colorless liquid or with little milk-like glisten ; should not be cloudy, impurity or with indiscernible deposit |
| 4. pH value | 6.5-7.5 |
| 5. Potency ( IU/dose ) | 80%-150% of indicated quantity ( $9\mu g$:$4.5 \times 10^6$IU, $15\mu g$: $7.5 \times 10^6$IU) |
| 6. Moisture | no more than 3.0% ( W/W) |

## 3. Experimental Content

Test samples at 2~8°C: The test samples were put into a 2~8°C refrigerator, then the above items of these samples were respectively tested in the 1st, 3rd, 6th, 9th, 12th, 18th, 24th, 30th, 36th month. The results were recorded.
Test samples at 25°C: The test samples were put into a thermostat at 25°C, then the above items of these samples were respectively tested in the 1st, 3rd, 6th, 9th, 12th, 18th, 24th, 30th month. The results were recorded.
Test samples at 37°C: The test samples were put into a thermostat at 37°C, then the above items of these samples were respectively tested in the 1st, 3rd, 6th, 9th, 12th, 18th, 24th month. The results were recorded.

## 4. Results and Conclusion

1) At 37°C, according to data collected at designated points during testing and compared with data before testing, the potency began descending from the 6th month and the changes in the three batches were similar. The appearance of other items had no changes.

2) At 25°C, according to data collected at designated points during testing and compared with data before the testing, the potency only had a little change, and the changes in the three batches were similar. The appearance of other items had no changes.

3) At 2-8°C, according to data collected at designated points during testing and compared with data before testing, the potency of the three batches all were stable. The appearance of other items also had no changes.

In conclusion, it is suggested that the lyophilized powder of recombinant super-compound interferon for injection should be better stored and transported at low temperatures. Without such conditions, the product can also be stored for short periods (i.e., 3 months) at room temperature.

**EXAMPLE 3.5**

**Production Flow Chart of rSIFN-co**

1. Production

1.1 Fermentation

Use mixture of LB+M9 as culturing medium. The amount of innoculum will be 1.5%. Agitate to OD600=0.4 (about 3.5 hours) under 32°C, then raise temperature to 42°C. Continue the agitation for another 6 hours, the expression of rSIFN-CO will reach the maximum level. The examination under scanning of the gel resulting from SDS-PAGE shows that the level of expression is up to 57%, which is the highest standard in China.

1.2 Purification

Centrifuge the bacteria solution to collect the bacterial pellet
↓
Physiological saline wash for two (2) times
↓
Adding buffer (50mM Tris-HCl, 1mM EDTA, 100mM NaCl, 1% Triton X-100, 1-2 M Urea), sonication to disrupt bacterial cells for 20-30 minutes
↓
Precipitate the buffer solution and wash a few times until the color turns into pure white
↓
Use 7M Guanidine HCl to denature
↓
Dilute the Guanidine HCl to renature, stay overnight
↓
Use Sephadex G25 to desalt
↓
Use 0.1 M NaCl to apply CM-Sepharose
↓
Do stepwise elution to collect the active peak ↓
After the active peak is desalted, apply to HPLC positively charged column
↓
Use 0.1 M NaCl to do stepwise elution, collect active peak which is the product of rSIFN-co ↓
Add protection carrier and lyophiling agent Separate lyophilized materials (rSIFN-co)

The purity of the product (rSIFN-co) from this production procedure is shown to 95% under the test of SDS-PAGE where molecular weight is 14.5 Kda. The reverse phase HPLC shows a single peak and the purity is up to 97%. Its specific activity is up to $1 \times 10^9$ IU/mg protein.

1.3 Packaging and Inspection

After HPLC purification, 2% human serum albumin, 1% sucrose and 1% glucose are added to the rSIFN-co. It is then separated and lyophilized into injection sample. When tested under the Wish-VVS inspection system, the result was $4.5 \times 10^8$ IU. When tested with aseptic inspection and pyrogen inspection under the standard requirement of China, the results were negative. This result complies with the requirements for IV injection.

2. Quality Control

2.1 Biological characteristics

(1) When using LB+M9 to cultivate bacteria, the characteristics should match with the typical characteristics of E-coli bacteria. No other bacteria were detected.
(2) When smeared for Gram staining and inspected under a microscope, it is bacteria-negative.
(3) Reaction to antibiotics is the same as those original bacteria.

(4) Electron microscope inspection shows typical characteristics of E-coli bacteria. No mycoplasma, virus spore or other micro pollutes was detected.
(5) Biochemical reaction test shows characteristics of E-coli bacteria.

2.2 Quality control of interferon expression

(1) Interferon expression (cultivated in an agitating platform) matches the amount of expression in original input bacteria.
(2) When tested with anti-interferon serum, a reaction is shown.
(3) Plasmid inspection: Restriction digest matched with the original plasmid.

2.3 Bacteria strain product

Bacteria strain product denotes the specimen from the original bacteria strain that was produced from the procedures shown in 1.2.
The bacteria strain product should be inspected as follows to make sure there is no derivation: Use LB to plate 2-3 pieces and cultivate. Separate and take 5-10 bacteria groups for the test of interferon expression. Repeat the test at least two (2) times. Only use the one which shows the highest % to be the bacteria strain product.

2.4 Innoculum

The innoculum denotes the chosen bacteria strain product after fermentation. The amount, cultivation time and most appropriate OD value of innoculum can be decided according to bacteria strain. An anti-polluted bacteria procedure should apply for whatever innoculum would be produced.

2.5 Growing of bacteria strain

Growing of bacteria strain would be done in a Bacteria Free room environment where no more than one bacterium is growing in the same room. Same culturing medium will be used for both bacteria strain and innoculum. The one used in rSIFN-co is LB.

2.6 Fermentation

(1) Fermentation only takes place in a clean fermentation room with a single bacteria fermentation environment.
(2) Cleaning of fermentation container and tube is done twice, before and after the insertion of culturing medium. Then, the container should be frozen to reach the appropriate temperature for innoculum.
(3) Avoid using antibiotic which might affect cell growth in the culturing medium.
(4) Fermentation parameters like temperature, pH value, dissolved oxygen and time required could be varied according to different types of bacterial strains.

2.7 Bacteria collection

(1) Centrifuge the bacteria solution to collect bacteria or use another method. All apparatus should be cleaned before and after the operation. The waste solution should be drained after the cleaning procedure.
(2) The bacteria should be kept under 4-8°C if they are going to be split within 24 hours. Otherwise, they should be kept under -30°C. Those are kept under such conditions can be used within 6 months.

2.8 Bacteria cell lysis

(1) Use appropriate buffer solution to balance the bacteria strain. Cell lysis can be done by physical, chemical or biological methods. Use centrifuge to precipitate the bacteria and apply cleaning solutions.
(2) If the chemical method is used to split cells, no solutions harmful to human beings should be used.

2.9 Purification

(1) Purification will get rid of most of the non-interferon contents. In the process of purification, no toxic materials should be found if extra elements are added.
(2) If using antibody affinity chromatography for purification, there should be an indication of the source and degree of purity. Also, inspection of small quality IgG should be performed.
(3) During the process of purification, clearance of pyrogen is critical. All apparatus should be checked to eliminate this interference.
(4) The highly concentrated interferon is known as "intermediate product". After inspection and tests, add albumin to raise the concentration to 2% which is now known as "albumin intermediate product". After examination and tests, it should be kept at -30°C and never thawed before use. This product should be used within 6 months.
(5) The albumin that is used in this process should also fulfill tests and requirements such as: negativity under RBSAG inspection and an indication of the ratio among monomer, dimer and polymer.

2.10 Production into tube product

(1) Filtration: Use 0.22 $\mu$ membrane to filter the bacteria. The product should be handled with aseptic techniques. Samples should be taken to test the value of the interferon.
(2) Dilution: Dilute the albumin intermediate product with 2% diluent. No preservative should be added. The product can be lyophilized after the aseptic inspection and pyrogen inspection.

2.11 Lyophilization

The lyophilization should not affect the activity of interferon, and the water content of said lyophilite will be maintained.

2.12 Inspection

There are two types of rSIFN-co made. One is for injection and the other for topical use. The specifications for the two are different. There are intermediate products and final products for each type. In the injection type, intermediate products include purified interferon, albumin intermediate product, and bacteria free albumin intermediate product. Final product from the injection type will denote only lyophilized product. The intermediate product in the topical type denotes only purified interferon. The final product from the topical type denotes only separated packed liquid formed lyophilized products.

2.13 Packaging

There is different packaging for the injection type and the topical type.

2.14 Storage

The product should be kept at 4°C. The purification solution should not be stored in a frozen state.

2.15 Expiration

The expiration period is two (2) years after the lyophilization procedure for lyophilized products. The expiration period is 6 months after individual packing for liquidated products.

**EXAMPLE 4**

**rSIFN-co inhibits HBV-DNA duplication and secretion of HBsAg and HBeAg.**

*Materials*

Solvent and Dispensing Method: Add 1ml saline into each vial, dissolve, and mix with MEM culture medium at different concentrations. Mix on the spot.

Control drugs: IFN-$\alpha$2b (Intron A) as lyophilized powder, purchased from Schering Plough. 3x10$^6$U each, mix to 3$\times$10$^6$IU/ml with culture medium; Infergen$^®$ (liquid solution), purchased from Amgen, 9$\mu$g, 0.3ml each, equal to 9X10$^6$IU, and mix with 9X10$^6$IU/ml culture medium preserve at 4°C; 2.2.15 cell: 2.2.15 cell line of hepatoma (Hep G2) cloned and transfected by HBV DNA, constructed by Mount Sinai Medical Center.

Reagent: MEM powder, Gibco American Ltd. cattle fetal blood serum, HycloneLab American Ltd. G-418(Geneticin); MEM dispensing, Gibco American Ltd.; L-Glutamyl, imported and packaged by JING KE Chemical Ltd.; HBsAg and HBeAg solid-phase radioimmunoassay box, Northward Reagent Institute of Chinese Isotope Ltd.; Biograncetina, Northern China Medicine; And Lipofectin, Gibco American Ltd.

Experimental goods and equipment: culture bottle, Denmark Tunclon™; 24-well and 96-well culture board, Corning American Ltd.; Carbon Dioxide hatching box, Shel-Lab American Ltd.; MEM culture medium 100ml: 10% cattle fetal blood serum, 3% Glutamyl1%, G418 380$\mu$g/ml, biograncetina50U/ml.

Method:

2.2.15 cell culture: Added 0.25% pancreatic enzyme into culture box with full of 2.2.15 cell, digest at 37°C for 3 minutes, and add culture medium to stop digest and disturb it to disperse the cells, reproduce with ratio of 1:3. They will reach full growth in 10 days.

Toxicity test: Set groups of different concentrations and a control group in which cells are not acted on with medicine. Digest cells, and dispense to a 100,000 cell/ml solution. Inoculate to 96-well culture board, 200$\mu$l each well, culture at 37°C for 24h with 5% $CO_2$. Test when simple cell layer grows.

Dispense rSIFN-co to 1.8x10$^7$IU/ml solution, then prepare a series of solutions diluted at two-fold gradients. Add into 96-well culture board, 3 wells per concentration. Change the solution every 4 days. Test cytopathic effect by microscope after 8 days. Fully destroy as 4, 75% as 3, 50% as 2, 25% as 1, zero as 0. Calculate average cell lesion and inhibition rate of different concentrations. Calculate TC50 and TC0 according to the Reed Muench method.

$$\text{TC50 = Antilog } \left(B + \frac{50-B}{A-B} \times C\right)$$

A=log >50% medicine concentration, B=log<50% medicine concentration, C=log dilution power

Inhibition test for HBeAg and HBsAg: Separate into positive and negative HBeAg and HBsAg contrast groups, cell contrast group and medicine concentration groups. Inoculate 700,000 cells/ml of 2.2.15 cell into 6-well culture board, 3 ml each well, culture at 37°C for 24h with 5% $CO_2$, then prepare 5 gradiently diluted solutions with 3-fold as the grade (Prepare 5 solutions, each with a different protein concentration. The concentration of Solution 2 is 3 times lower than that of Solution 1, the concentration of Solution 3 is 3 times lower than that of Solution 2, etc.) 4.5$\times$10$^6$IU/ml, 1.5$\times$10$^6$IU/ml, 0.5$\times$10$^6$IU/ml, 0.17$\times$10$^6$1U/ml, and 0.056x10$^6$1U/ml, 1 well per concentration, culture at 37°C for 24h with 5% $CO_2$. Change solutions every 4 days using the same solution. Collect all culture medium on the 8th day. Preserve at -20 °C Repeat test 3 times to estimate HBsAg and HBeAg with solid-phase radioimmunoassay box (Northward Reagent Institute of Chinese Isotope Ltd.). Estimate cpm value of each well with a $\gamma$- accounting machine.

Effects calculation: Calculate cpm mean value of contrast groups and different-concentration groups and their standard deviation, P/N value such as inhibition rate, IC50 and SI.

1)

$$\text{Antigen inhibition rate (\%) } = \frac{A-B}{A} \times 100$$

A = cpm of control group; B = cpm of test group;
2) Counting the half-efficiency concentration of the medicine

$$\texttt{Antigen inhibition IC50 = Antilog}\ (\texttt{B} + \frac{50-B}{A-B} \times \texttt{C})$$

A=log>50% medicine concentration, B=log<50% medicine concentration, C=log dilution power

3) SI of interspace-conformation changed rSIFN-co effect on HBsAg and HBeAg in 2.2.15 cell culture:

$$SI = \frac{TC50}{IC50}$$

4) Estimate the differences in cpm of each dilution degree from the control group using student t test

Southern blot: (1) HBV-DNA extract in 2.2.15 cell: Culture cell 8 days. Exsuction culture medium (Separate cells from culture medium by means of draining the culture medium.). Add lysis buffer to break cells, then extract 2 times with a mixture of phenol, chloroform and isoamyl alcohol (1:1:1), 10,000g centrifuge. Collect the supernatant adding anhydrous alcohol to deposit nucleic acid. Vacuum draw, redissolve into $20\mu l$TE buffer. (2) Electrophoresis: Add 6XDNA loading buffer, electrophoresis on 1.5% agarose gel, IV/cm, at fixed pressure for 14-18h. (3) Denaturation and hybridization: respectively dip gel into HCl, denaturaion buffer and neutralization buffer. (4) Transmembrane: Make an orderly transfer of DNA to Hybond-N membrane. Bake, hybridize and expose with dot blot hybridization. Scan and analyze relative density with gel-pro software. Calculate inhibition rate and IC50.

Results

Results from Tables 4.1, 4.2 and 4.3 show: After maximum innocuous concentration exponent culturing for 8 days with 2.2.15 cell, the maxima is $9.0 \pm 0 \times 10^6$IU/ml average inhibition rate of maximum innocuous concentration rSIFN-co to HBeAg is $46.0 \pm 5.25\%$ (P<0.001), IC50 is $4.54 \pm 1.32$X$10^6$IU/ml, SI is 3.96; rate to HBsAg is $44.8 \pm 6.6\%$, IC50 is $6.49 \pm 0.42 \times 10^6$IU/ml, SI is 2.77. This shows that rSIFN-co can significantly inhibit the activity of HBeAg and HBsAg, but that the IFN of the contrast group and Infergen® cannot. It has also been proven in clinic that rSIFN-co can decrease HBeAg and HBsAg or return them to normal levels.

Table 4.1: Results of inhibition rate of rSIFN-co to HBsAg and HBeAg

| First batch: (rSIFN-co) | | | | | | | | | | |
|---|---|---|---|---|---|---|---|---|---|---|
| Inhibition effect to HBeAg | | | | | | | | | | |
| Concentration ($\times 10^4$IU/ml) | First well | Second well | Third well | Inhibition rate | | | Average inhibition rate | Accumulation | 1-Accumulation | Accumulated inhibition rate |
| | | | | First well | Second well | Third well | | | | |
| 900 | 9026 | 8976 | 10476 | 0.436227 | 0.43935 | 0.345659 | 0.407079 | 0.945909 | 0.592921 | 0.614693546 |
| 300 | 9616 | 12082 | 10098 | 0.3993754 | 0.245347 | 0.369269 | 0.337997 | 0.5388299 | 1.254924 | 0.300392321 |
| 100 | 9822 | 16002 | 12800 | 0.386508 | 0.0005 | 0.2005 | 0.195836 | 0.200833 | 2.059088 | 0.08867188 |
| 33.33333 | 15770 | 19306 | 16824 | 0.014991 | 0 | 0 | 0.004997 | 0.0049969 | 3.054091 | 0.001633453 |
| 11.11111 | 19172 | 22270 | 18934 | 0 | 0 | 0 | 0 | 0 | 4.054091 | 0 |
| Control | Cell | 16010 | | Blank | 0 | | Dilution | 3 | IC50 | 602.74446016 |
| Inhibition effect to HBsAg | | | | | | | | | | |
| Concentration ($\times 10^4$IU/ml) | First well | Second well | Third well | Inhibition rate | | | Average inhibition rate | Accumulation | 1-Accumulation | Accumulated inhibition rate |
| | | | | First well | Second well | Third well | | | | |
| 900 | 7706 | 7240 | 7114 | 0.342155 | 0.381936 | 0.392693 | 0.372261 | 0.922258 | 0.627739 | 0.595006426 |
| 300 | 8856 | 7778 | 9476 | 0.2439816 | 0.336008 | 0.191053 | 0.257014 | 0.5499972 | 1.370724 | 0.286349225 |
| 100 | 10818 | 10720 | 10330 | 0.07649 | 0.084856 | 0.118149 | 0.093165 | 0.292983 | 2.27756 | 0.113977019 |
| 33.33333 | 10744 | 11114 | 10570 | 0.082807 | 0.051221 | 0.097661 | 0.07723 | 0.1998179 | 3.20033 | 0.058767408 |
| 11.11111 | 10672 | 9352 | 10810 | 0.088953 | 0.201639 | 0.077173 | 0.122588 | 0.122588 | 4.077742 | 0.02918541 |
| Control | Cell | 11714 | | Blank | 0 | | Dilution | 3 | IC50 | 641.7736749 |
| Second batch: (rSIFN-co) | | | | | | | | | | |
| Inhibition effect to HBeAg | | | | | | | | | | |
| Concentration ($\times 10^4$IU/ml ) | First well | Second well | Third well | Inhibition rate | | | Average inhibition rate | Accumulation | 1-Accumulation | Accumulated inhibition rate |
| | | | | First well | Second well | Third well | | | | |
| 900 | 7818 | 8516 | 9350 | 0.554378 | 0.514592 | 0.467054 | 0.512008 | 1.371181 | 0.487992 | 0.737521972 |

(continued)

| Second batch: (rSIFN-co) | | | | | | | | | | |
|---|---|---|---|---|---|---|---|---|---|---|
| **Inhibition effect to HBeAg** | | | | | | | | | | |
| Concentration (×10⁴IU/ml ) | First well | Second well | Third well | Inhibition rate | | | Average inhibition rate | Accumulation | 1-Accumulation | Accumulated inhibition rate |
| | | | | First well | Second well | Third well | | | | |
| 300 | 10344 | 10628 | 9160 | 0.4103967 | 0.394209 | 0.477884 | 0.427497 | 0.8591731 | 1.060996 | 0.447563245 |
| 100 | 12296 | 14228 | 13262 | 0.299134 | 0.18901 | 0.244072 | 0.244072 | 0.4316522 | 1.816423 | 0.19201839 |
| 33.33333 | 15364 | 17414 | 16188 | 0.124259 | 0.00741 | 0.77291 | 0.069653 | 0.1876045 | 2.74677 | 0.063933386 |
| 11.11111 | 17386 | 13632 | 15406 | 0.009006 | 0.222982 | 0.121865 | 0.117951 | 0.117951 | 3.628819 | 0.03148073 |
| Control | Cell | 16962 | | Blank | 0 | | Dilution | 3 | IC50 | 365.9357846 |
| **Inhibition effect to HBsAg** | | | | | | | | | | |
| Concentration (×10⁴IU/ml) | First well | Second well | Third well | Inhibition rate | | | Average inhibition rate | Accumulation | 1-Accumulation | Accumulated inhibition rate |
| | | | | First well | Second well | Third well | | | | |
| 900 | 5784 | 6198 | 5792 | 0.498265 | 0.462353 | 0.497571 | 0.486063 | 0.893477 | 0.513937 | 0.634835847 |
| 300 | 7150 | 8534 | 8318 | 0.379771 | 0.259715 | 0.278452 | 0.30598 | 0.4074138 | 1.207957 | 0.252210647 |
| 100 | 9830 | 11212 | 10210 | 0.147294 | 0.027412 | 0.11433 | 0.096345 | 0.101434 | 2.111612 | 0.04583464 |
| 33.33333 | 13942 | 12368 | 13478 | 0 | 0 | 0 | 0 | 0.0050891 | 3.111612 | 0.001632835 |
| 11.11111 | 12418 | 11634 | 11352 | 0 | 0 | 0.015267 | 0.005089 | 0.005089 | 4.106523 | 0.001237728 |
| Control | Cell | | | Blank | 0 | Dilution | Dilution | 3 | IC50 | 611.0919568 |
| Third batch: (rSIFN-co) | | | | | | | | | | |
| **Inhibition effect to HBeAg** | | | | | | | | | | |
| Concentration × 10⁴IU/ml) | First well | Second well | Third well | Inhibition rate | | | Average inhibition rate | Accumulatio n | 1-Accumulat ion | Accumulated inhibition rate |
| | | | | First well | Second well | Third well | | | | |
| 900 | 9702 | 9614 | 8110 | 0.428016 | 0.433204 | 0.52187 2 | 0.461031 | 1.316983 | 0.538969 | 0.709599543 |
| 300 | 8914 | 10032 | 8870 | 0.4744723 | 0.40856 | 0.47706 6 | 0.453366 | 0.8559525 | 1.085603 | 0.440859127 |

EP 2 325 202 B1

| Third batch: (rSIFN-co) | | | | | | | | | | |
|---|---|---|---|---|---|---|---|---|---|---|
| Inhibition effect to HBeAg | | | | | | | | | | |
| Concentration × 10⁴IU/ml) | First well | Second well | Third well | Inhibition rate | | | Average inhibition rate | Accumulatio n | 1-Accumulat ion | Accumulated inhibition rate |
| | | | | First well | Second well | Third well | | | | |
| 100 | 16312 | 12688 | 13934 | 0.038321 | 0.251975 | 0.17851 7 | 0.156271 | 0.402586 | 1.929332 | 0.172691621 |
| 33.33333 | 15080 | 12814 | 13288 | 0.110954 | 0.244547 | 0.21660 2 | 0.190701 | 0.2463153 | 2.738631 | 0.082519158 |
| 11.11111 | 21928 | 15366 | 15728 | 0 | 0.094093 | 0.07275 1 | 0.0055615 | 0.055615 | 3.683017 | 0.014875633 |
| Control | Cell | 17544 | | Blank | 0 | | Dilution | 3 | IC50 | 382.0496935 |
| | Inhibition effect to HBsAg | | | | | | | | | |
| Concentration (× 10⁴IU/ml) | First well | Second well | Third well | Inhibition rate | | | Average inhibition rate | Accumulatio n | 1-Accumulat ion | Accumulated inhibition rate |
| | | | | First well | Second well | Third well | | | | |
| 900 | 5616 | 6228 | 5346 | 0.496864 | 0.442035 | 0.52105 4 | 0.486651 | 0.763125 | 0.513349 | 0.597838293 |
| 300 | 8542 | 8590 | 7096 | 0.234725 | 0.230425 | 0.36427 2 | 0.276474 | 0.2764738 | 1.236875 | 0.182690031 |
| 100 | 11420 | 11360 | 11394 | 0 | 0 | 0 | 0 | 0 | 2.236875 | 0 |
| 33.33333 | 12656 | 11582 | 13110 | 0 | 0 | 0 | 0 | 0 | | 0 |
| 11.11111 | 13142 | 12336 | 13342 | 0 | 0 | 0 | 0 | 0 | 4.236875 | 0 |
| Control | Cell | 11528 | | Blank | 0 | | Dilution | 3 | IC50 | 694.7027149 |
| HBeAg: Average IC50: 450.2434 SD: 132.315479 HBsAg:Average IC50: 649.1894 SD: 42.29580 | | | | | | | | | | |

EP 2 325 202 B1

Table 4.2: Results of inhibition rate of Intron A(IFN-α2b) to HBsAg and HBeAg

| Inhibition effect to HBeAg | | | | | | | | | | |
|---|---|---|---|---|---|---|---|---|---|---|
| Concentration (×10⁴IU/ml) | First well | Second well | Third well | Inhibition rate | | | Average inhibition rate | Accumulation | 1-Accumulation | Accumulated inhibition rate |
| | | | | First well | Second well | Third well | | | | |
| 300 | 14918 | 11724 | 9950 | 0 | 0.029711 | 0.176529 | 0.068747 | 0.068747 | 0.931253 | 0.068746724 |
| 100 | 14868 | 16890 | 15182 | 0 | 0 | 0 | 0 | 0 | 1.931253 | 0 |
| 33.33333 | 16760 | 21716 | 16400 | 0 | 0 | 0 | 0 | 0 | 2.931253 | 0 |
| 11.11111 | 20854 | 15042 | 16168 | 0 | 0 | 0 | 0 | 0 | 3.931253 | 0 |
| 3.703704 | 12083 | 12083 | 12083 | 0 | 0 | 0 | 0 | 0 | 4.931253 | 0 |
| Control | Cell | 17544 | | Blank | 0 | | Dilution | 3 | IC50 | FALSE |

| Inhibition effect to HBsAg | | | | | | | | | | |
|---|---|---|---|---|---|---|---|---|---|---|
| Concentration (×10⁴IU/ml) | First well | Second well | Third well | Inhibition rate | | | Average inhibition rate | Accumulation | 1-Accumulation | Accumulated inhibition rate |
| | | | | First well | Second well | Third well | | | | |
| 300 | 9226 | 8196 | 9658 | 0.152489 | 0.247106 | 0.521054 | 0.1708 | 0.189295 | 0.8292 | 0.185857736 |
| 100 | 10946 | 10340 | 10828 | 0 | 0.050156 | 0.364272 | 0.018495 | 0.0184947 | 1.810705 | 0.010110817 |
| 33.33333 | 12250 | 12980 | 13934 | 0 | 0 | 0 | 0 | 0 | 2.810705 | 0 |
| 11.11111 | 12634 | 12342 | 12000 | 0 | 0 | 0 | 0 | 0 | 3.810705 | 0 |
| 3.703704 | 10886 | 10886 | 10886 | 0 | 0 | 0 | 0 | 0 | 4.810705 | 0 |
| Control | Cell | 10886 | | Blank | 0 | | Dilution | 3 | IC50 | FALSE |

Table 4.3: Results of inhibition rate of Infergen® to HBsAg and HBeAg

**First batch: (Infergen®)**

**Inhibition effect to HBeAg**

| Concentration (×10⁴IU/ml) | First well | Second well | Third well | Inhibition rate | | | Average rate inhibition rate | Accumulation | 1-Accumulation | Accumulated inhibition rate |
| --- | --- | --- | --- | --- | --- | --- | --- | --- | --- | --- |
| | | | | First well | Second well | Third well | | | | |
| 900 | 14172 | 12156 | 17306 | 0.091655 | 0.220869 | 0 | 0.104175 | 0.306157 | 0.895825 | 0.254710274 |
| 300 | 13390 | 12288 | 16252 | 0.1417767 | 0.212409 | 0 | 0.118062 | 0.2019827 | 1.777764 | 0.102024519 |
| 100 | 14364 | 18834 | 14194 | 0.079349 | 0 | 0.090245 | 0.056531 | 0.083921 | 2.721232 | 0.029916678 |
| 33.33333 | 15722 | 16034 | 16340 | 0 | 0 | 0 | 0 | 0.0273897 | 3.721232 | 0.007306592 |
| 11.11111 | 17504 | 17652 | 14320 | 0 | 0 | 0.082169 | 0.02739 | 0.02739 | 4.693843 | 0.005801377 |
| Control | Cell | 15602 | | Blank | 0 | | Dilution | 3 | IC50 | FALSE |

**Inhibition effect to HBsAg**

| Concentration (×10⁴IU/ml) | First well | Second well | Third well | Inhibition rate | | | Average inhibition rate | Accumulation | 1-Accumulation | Accumulated inhibition rate |
| --- | --- | --- | --- | --- | --- | --- | --- | --- | --- | --- |
| | | | | First well | Second well | Third well | | | | |
| 900 | 12080 | 11692 | 12234 | 0 | 0.01275 | 0 | 0.00425 | 0.025163 | 0.99575 | 0.029697111 |
| 300 | 12840 | 11484 | 12350 | 0 | 0.030313 | 0 | 0.010104 | 0.0209125 | 1.985646 | 0.010422073 |
| 100 | 12894 | 14696 | 15086 | 0 | 0 | 0 | 0 | 0.010808 | 2.985646 | 0.003606955 |
| 33.33333 | 15032 | 12928 | 13020 | 0 | 0 | 0 | 0 | 0.0108081 | 3.985646 | 0.002704416 |
| 11.11111 | 11794 | 11984 | 11508 | 0.004137 | 0 | 0.028287 | 0.010808 | 0.010808 | 4.974837 | 0.002167838 |
| Control | Cell | 11843 | | Blank | 0 | | Dilution | 3 | IC50 | FALSE |

**Second batch: (Infergen®)**

**Inhibition effect to HBeAg**

| Concentration (×10⁴IU/ml) | First well | Second well | Third well | Inhibition rate | | | Average inhibition rate | Accumulation | 1-Accumulation | Accumulated inhibition rate |
| --- | --- | --- | --- | --- | --- | --- | --- | --- | --- | --- |
| | | | | First well | Second well | Third well | | | | |
| 900 | 6278 | 6376 | 6408 | 0.200051 | 0.187564 | 0.183486 | 0.190367 | 0.274635 | 0.809633 | 0.253290505 |

EP 2 325 202 B1

(continued)

**Second batch: (Infergen®)**

Inhibition effect to HBeAg

| Concentration (×10⁴IU/ml) | First well | Second well | Third well | Inhibition rate First well | Inhibition rate Second well | Inhibition rate Third well | Average inhibition rate | Accumulation | 1-Accumulation | Accumulated inhibition rate |
|---|---|---|---|---|---|---|---|---|---|---|
| 300 | 7692 | 9092 | 6394 | 0.0198777 | 0 | 0.18527 | 0.068383 | 0.0842678 | 1.74125 | 0.046161005 |
| 100 | 8960 | 7474 | 8190 | 0 | 0.047655 | 0 | 0.015885 | 0.015885 | 2.725365 | 0.005794856 |
| 33.33333 | 8530 | 8144 | 9682 | 0 | 0 | 0 | 0 | 0 | 3.725365 | 0 |
| 11.11111 | 7848 | 7848 | 7848 | 0 | 0 | 0 | 0 | 0 | 4.725365 | 0 |
| Control | Cell 7848 | 7848 | | Blank 0 | | | Dilution 0 | 3 | IC50 | FALSE |

Inhibition effect to HBsAg

| Concentration (×10⁴IU/ml) | First well | Second well | Third well | Inhibition rate First well | Inhibition rate Second well | Inhibition rate Third well | Average inhibition rate | Accumulation | 1-Accumulation | Accumulated inhibition rate |
|---|---|---|---|---|---|---|---|---|---|---|
| 900 | 12364 | 12268 | 12274 | 0.036171 | 0.043655 | 0.043187 | 0.041004 | 0.140162 | 0.958996 | 0.12751773 |
| 300 | 11590 | 12708 | 13716 | 0.0965076 | 0.009355 | 0 | 0.035287 | 0.0991581 | 1.923709 | 0.0490186 |
| 100 | 12448 | 13468 | 13982 | 0.029623 | 0 | 0 | 0.009874 | 0.063871 | 2.913834 | 0.02144964 |
| 33.33333 | 12616 | 11346 | 12444 | 0.016526 | 0.115529 | 0.029935 | 0.053996 | 0.0539965 | 3.859838 | 0.013796309 |
| 11.11111 | 12828 | 12828 | 12828 | 0 | 0 | 0 | 0 | 0 | 4.859838 | 0 |
| Control | Cell 12828 | 12828 | | Blank 0 | | | Dilution 0 | 3 | IC50 | FALSE |

**Third batch: (Infergen®)**

Inhibition effect to HBeAg

| Concentration (×10⁴IU/ml) | First well | Second well | Third well | Inhibition rate First well | Inhibition rate Second well | Inhibition rate Third well | Average inhibition rate | Accumulation | 1-Accumulation | Accumulated inhibition rate |
|---|---|---|---|---|---|---|---|---|---|---|
| 900 | 7240 | 6642 | 6158 | 0.064599 | 0.14186 | 0.204393 | 0.136951 | 0.217399 | 0.863049 | 0.201211735 |
| 300 | 11072 | 8786 | 6902 | 0 | 0 | 0.108269 | 0.03609 | 0.0804479 | 1.82696 | 0.042176564 |

(continued)

| Third batch: (Infergen®) | | | | | | | | | | |
|---|---|---|---|---|---|---|---|---|---|---|
| Inhibition effect to HBeAg | | | | | | | | | | |
| Concentration ($\times 10^4$IU/ml) | First well | Second well | Third well | Inhibition rate | | | Average inhibition rate | Accumulation | 1-Accumulation | Accumulated inhibition rate |
| | | | | First well | Second well | Third well | | | | |
| 100 | 7016 | 9726 | 7552 | 0.09354 | 0 | 0.024289 | 0.039276 | 0.044358 | 2.787683 | 0.015663017 |
| 33.33333 | 7622 | 8866 | 8676 | 0.015245 | 0 | 0 | 0.005082 | 0.0050818 | 3.782601 | 0.001341671 |
| 11.11111 | 7740 | 7740 | 7740 | 0 | 0 | 0 | 0 | 0 | 4.782601 | 0 |
| Control | Cell | 7740 | | Blank | 0 | | Dilution | 3 | IC50 | FALSE |
| Inhibition effect to HBsAg | | | | | | | | | | |
| Concentration ($\times 10^4$IU/ml) | First well | Second well | Third well | Inhibition rate | | | Average inhibition rate | Accumulation | 1-Accumulation | Accumulated inhibition rate |
| | | | | First well | Second well | Third well | | | | |
| 900 | 11048 | 11856 | 11902 | 0.04775 | 0 | 0 | 0.015917 | 0.015917 | 0.984083 | 0.015916796 |
| 300 | 13454 | 12896 | 11798 | 0 | 0 | 0 | 0 | 0 | 1.984083 | 0 |
| 100 | 12846 | 13160 | 12546 | 0 | 0 | 0 | 0 | 0 | 2.984083 | 0 |
| 33.33333 | 12680 | 12458 | 12360 | 0 | 0 | 0 | 0 | 0 | 3.984083 | 0 |
| 11.11111 | 11602 | 11602 | 11602 | 0 | 0 | 0 | 0 | 0 | 4.984083 | 0 |
| Control | Cell | 11602 | | Blank | 0 | | Dilution | 3 | IC50 | FALSE |
| HBeAg: Average IC50: 0 SD: 0<br>HBsAg: Average IC50: 0 SD: 0 | | | | | | | | | | |

## EXAMPLE 5

### Preparation of rSIFN-co

Preparation of lyophilized injection

|  | Lyophilized powder |
|---|---|
| Stock Solution of rSIFN-co | 34.5 $\mu$g/ml |
| PB (pH7.0) | 10mmol/L |
| Glycine | 0.4mol/L |

Preparation technique: Weigh materials according to recipe. Dissolve with sterile and pyrogen-free water. Filter through 0.22$\mu$m membrane to de-bacterialize, preserve at 6-10°C. Fill in vials after affirming they are sterile and pyrogen-free, 0.3 ml /vial or 0.5 ml/vial, and lyophilize in freeze dryer.

Preparation of liquid injection

|  | Solution |
|---|---|
| Stock Solution of rSIFN-co | 34.5 $\mu$g/ml |
| PB (pH7.0) | 25mmol/L |
| NaCl | 0.1mol/L |

Preparation: Weigh materials according to recipe. Add to desired level with sterile and pyrogen-free water. Filter through 0.22$\mu$m membrane to de-bacterialize, preserve at 6-10°C. Fill in airtight vial after affirming it is sterile and non-pyrogen at 0.3 ml /vial or 0.5 ml/vial. Store at 2-10°C, and protect from light.

## EXAMPLE 6

### Acute Toxicity of rSIFN-co

Treat mice with large dose (150$\mu$g/kg, equal to 1000 times of the normal dose per kilo used in treatment of adult patients) of rSIFN-co at one time by intramuscular injection. Then observe and record their deaths and toxic reactions. Results show that: 24 hours after injection, no abnormal reaction had been recorded. The organs of the animals which had been selected to be killed also had no signs of abnormal changes. Those remaining mice were all kept alive and were normal after two weeks. The weights of mice in the experimental group and control group all increased, and the ratio of increase showed no obvious difference between the two groups (P>0.05) according to their weights on the fourteenth day. No abnormal changes were seen from the main organs of those mice after two weeks.

1. Experimental material

1.1 Animals

40 healthy adult mice, weighing 18-22g, half male and half female, qualified by Sichuan experiment animal control center.

1.2 Medicines

rSIFN-co (Provided by Sichuan Huiyang Life-engineering Ltd.) sterilized solution, 0.15 mg/ml, Lot: 981201 rSIFN-co was administered *i.m.* in saline.

2. Method

Separate the 40 mice into two groups randomly, one for experimental medicine, another for control. Inject medicines or saline at the same ratio (0.1 ml/10 g) through muscle to each mouse according to which group they belong. (150 $\mu$g/kg of rSIFN-co for experimental group; and saline for control group). After injection, observe and record acute toxicity shown in mice. Kill half of the mice (male and female each half) to check whether there were any abnormal pathologic changes in their main organs, such as heart, spleen, liver, lung, kidney, adrenal gland, stomach, duodenum, etc. after 24 hours. Those that remain are kept and observed until the fourteenth day. Weigh all mice, kill them, and then observe the appearance of the organs listed above to see if there are any abnormalities. Take pathological tissue and examine it, using the examination to assess the difference in weight increases in the two groups.

3. Results

Results show that there was no acute toxicity seen after all mice were treated with i.m. rSIFN-co with 150 $\mu$g/kg at a time, equal to 1000 times the normal dose per kilo used in treatment of adult patients. In the 14 days after injection, all mice lived well. They ate, drank, exercised, and excreted normally and showed normal hair conditions. None of them died. The observation of the main organs of the randomly selected mice shows no abnormal changes 24 hours after injection. 14 days after injection, all remaining mice were killed. Autopsies also showed no changes. The weights of mice in the two groups all increased, but no obvious difference was shown when accessed with statistic method ($p >$ 0.05). See Table 6.1:

Table 6.1 Influence to weights of mice after injection of rSIFN-co

| Group | Dose | Animal | Weights before injection (g) | Weights after injection (g) | Increased value of weights (g) |
|-------|------|--------|------------------------------|------------------------------|--------------------------------|
| Control | 0 | 20 | 19.8 ± 1.7 | 30.8 ± 2.8 | 11.0 ± 2.9 |
| rSIFN-co | 150 | 20 | 19.4 ± 1.7 | 32.1 ± 3.3 | 12.7 ± 4.3 |

4. Conclusion

Under conditions of this experiment, there were no toxic reactions in all mice after injection of rSIFN-co with 150 $\mu$g/kg. The conclusion can be reached that the maximum tolerable dose of i.m. in mice is 150 $\mu$g/kg, which is equal to 1000 times the normal dose per kilo used in treatment of adult patients.

**EXAMPLE 7**

**The clinic effects of recombinant super-compound interferon (rSIFN-co)**

The recombinant super-compound interferon (rSIFN-co) is an invention for viral disease therapy, especially for hepatitis. Meanwhile, it can inhibit the activity of EB viruses, VSV, Herpes simplex viruses, cornaviruses, measles viruses, et al. Using Wish cells /VSV system as the assay for anti-virus activity, the results showed that: the other rIFN, was $0.9 \times 10^8$ IU/mg, Intron A was $2.0 \times 10^8$ IU/mg and rSIFN-co was $9 \times 10^8$ IU/mg. The anti-viral activity of rSIFN-co is much higher than those of the former two.

Under the permission of the State Food and Drug Administration (SFDA), People's Republic of China, the clinical trials have taken place in West China Hospital, Sichuan University, the Second Hospital of Chongqing Medical University, the First Hospital of School of Medical, Zhejiang University since the February 2003. The clinical treatment which focuses on hepatitis B is conducted under the guidance of the mutilcenter, double-blind random test. IFN-$\alpha$1b was used as control, and the primary results showed the following:

**The effect of rSIFN-co compared with IFN-$\alpha$1b in the treatment of chronic active hepatitis B**

**1. Standard of patients selection:**

Standards 1-4 are effective for both treatment with rSIFN-co (9$\mu$g) and IFN-$\alpha$1b (5MU, 50$\mu$g), and Standard 1-5 are for rSIFN-co (15$\mu$g) treatment.

   1). Age: 18-65

2). HBsAg-test positive over last six months, HBeAg-test positive, PCR assay, HBV-DNA copies $\geq 10^5$/ml

3). ALT $\geq$ two times the normal value

4). Never received IFN treatment; or received the Lamividine treatment but failed or relapsed

5) Once received other IFNs (3MU or 5MU) treatment six months ago following the standard of SFDA, but failed or relapsed

**2. Evaluation of the effects:**

In reference to the recommendations from the Tenth China National Committee of Virus Hepatitis and Hepatopathy, the effects were divided into three degrees according to the ALT level, HBV-DNA and HBeAg tests.

Response: ALT normal level, HBV-DNA negative, HBeAg negative

Partial response: ALT normal level, HBV-DNA or HBeAg negative

Non response: ALT, HBV-DNA and HBeAg unchanged

The response and partial response groups were considered effective cases.

**3. Results of clinic trial:**

Group A: treatment with rSIFN-co (9μg)

Group B: treatment with IFN-α1b (5MU, 50 μg)

| Period | group | Medicine | cases | Effective Rate | HBsAg Transfer to negative rate | HBeAg Transfer to negative rate | HBV-DNA Transfer to negative rate | Heptal function Recovery rate |
|--------|-------|----------|-------|----------------|-------------------------------|-------------------------------|----------------------------------|-------------------------------|
| 8-12 week | A | rSIFN-co (9μg) | 32 | 46.88 (15) | 9.38 (3) | 28.12 (9) | 37.50 (12) | 84.38 (27) |
| | B | IFN-α1b (5MU, 50 μg) | 32 | 21.88 (7) | 0.00 (0) | 9.38 (3) | 15.62 (5) | 56.25 (18) |
| 16-24 week | A | rSIFN-co(9μg) | 64 | 54.69 (35) | 7.81 (5) | 25.00 (16) | 34.38 (22) | 90.62 (58) |
| | B | IFN-α1b (5MU, 50 μg) | 64 | 25.00 (16) | 0.00 (0) | 9.38 (6) | 18.75 (12) | 78.13 (50) |

In Group C, the cases were prior treatment of chronic active hepatitis B with other IFNs (3MU or 5MU) that failed or relapsed and then were treated with rSIFN-co (15 μg), subcutaneous injection, every one day, for 24 weeks. The total cases were 13. After 12 weeks treatment, 7 of 13 (53.85%) were effective. 3 of 13 (23.08%) HBeAg transferred to negative; 7 of 13(53.85%) HBV-DNA transferred to negative; 11 of 13 (84.62%) heptal functions recovered to normal.

**4. The side effects of rSIFN-co compared with IFN-α1b in the treatment**

The side effects of IFN include fever, nausea, myalgia, anorexia, hair loss, leucopenia and thrombocytopenia, etc. The maximum dose of IFN-α1b is 5MIU per time; the routine dose is 3 MIU. When taken the routine dose, 90% patients have I- II degree (WHO standard) side effects. They had fever lower than 38°C, nausea, myalgia, anorexia, etc. When taken at maximum dose, the rate of side effects did not rise obviously, but were more serious. The maximum dose of rSIFN-co is 24µg, subcutaneous injection, every one day for 3 months. The routine dose is 9µg. When routine doses were used, less than 50% of patients had I-II degree (WHO standard) side effects, including fever below 38°C, nausea, myalgia, anorexia, leucopenia and slight thrombocytopenia. With maximum dosage, about 50% patients suffered from leucopenia and thrombocytopenia after using **rSIFN-co** one month, but those side effects disappeared after stopping treatment for one week. It is safe for continued use.

**The observations of rSIFN-co treat hepatitis C**

**1. Standard of patients selection**

1) age: 18-65
2) HCV antibody positive
3) ALT≥1.5 times of the normal value, last more than 6 months

**2. Evaluation of the effects:**

Referring to the standard of Infergen® for treatment of hepatitis C and according to the ALT level and HCV-RNA test, divided the effects into three degree:

Response: ALT normal level, HCV-RNA negative
Partial response: ALT normal level, HCV-RNA unchanged
Non response: ALT and HCV-RNA unchanged

**3. Effects in clinic**

The clinical trial was done at the same time with hepatitis B treatment. 46 cases received the treatment, 9 µg each time, subcutaneous injection, every day for 24 weeks. After treatment, 26 of 46 (56.52%) have obvious effects, 12 of 46 (26.08%) HCV-RNA transferred to negative, 26 of 46 (56.52%) heptal functions recovered to normal.

**EXAMPLE 8**

**Recombinant Super-Compound Interferon Spray**

**Major component:** Recombinant Super Compound Interferon **Characteristic:** Liquid, no insoluble material
**Pharmacology:** Recombinant Super-Compound Interferon has a wide spectrum of anti-virus activity. Its effects are 5-20 times higher than those interferons (IFNs) which are available on the market. It can inhibit coronavirus growth in cell culture. The mechanism is interruption of the combination reaction between the IFN and the correspondent receptor, and inducement of the expression of 2'5'-A synthesizenzyme, protein kinase R in the target cell, therefore inhibiting expression of the viral protein. IFN can induce expression of various anti-virus proteins to inhibit the reproduce of viral proteins, enhance the function of Natural Killer (NK) cell and other Immune regulative functions, and inhibit the invasion of viruses.
**Acute toxicity:** All mice are alive after the maximum dose (1000 times to human dose) subcutaneous injection, did not observe LD50.
**Indication:** Prevention of Severe Acute Respiratory Syndrome **Dosage and Administration:**Spray to both nasal cavity and throat, three times a day.
**Adverse reactions:** There was no report of adverse reactions from the rIFN spray. It did not induce allergy. If the stimulation is occasional, adverse gastrointestinal reaction is small, and no other obvious adverse reaction was noted during treatment, it is safe to continue use. All reactions will resolve themselves.
**Warning:** Patients allergic to αIFN and productions of *E. Coli.* cannot use this product.
**Precautions:** Before first use, spray twice to expel the air. If there is any cloudy precipitation material, if the product is expired, or there is material on the vial, do not use it.
**Pediatric Use:** It is unclear.
**Geriatric Use:** It is unclear.
**Nursing mothers and pregnant women:**Forbidden

**Drug Interactions:** It is unclear.

**Overdose:** Excess 150 ug ($7.5 \times 10^7$ IU) each time, fever, anorexia, myalgia, chill will happen more frequently. There is no severe adverse reaction.

**Supplied:** 1 spray/ pack, 20 ug ($1 \times 10^7$ IU)/3ml

**Storage:** Store at 4-8°C. Do not freeze, protect from light.

**Effective period:** Approximately one year

**Manufacture:** Manufactured by Sichuan Huiyang life-engineering Ltd.

**Address:** 8 Yusa Road, Room 902, Building A

Chengdu, 610017

Sichuan, P.R. China


## EXAMPLE 9-A

### In vitro effect of a new-style recombinant compound interferon on SARS-associated coronavirus

**Sample supplied by:** Huiyang Life Engineering Lt Company, SiChuan Province

**Experimenter:** Molecular Biology Department, microorganism and epidemiology Institute, Academy of Military Medical Science

**Original data:** Preserved in archive of Molecular Biology Department, microorganism and epidemiology Institute, Academy of Military Medical Science

### 1. Materials

**Medicine:** New-type recombinant compound interferon, $9\mu$g each, supplied by Huiyang Life Engineering Lt Company, SiChuan Province, Lot number: 20020501.

**Cells:** Vero $E_6$, supplied by Molecular Biology Department of Microorganism and Epidemiology Institute, Academy of Military Medical Science.

**Virus:** SARS-associated coronavirus, BJ-01, supplied by Molecular Biology Department of Microorganism and Epidemiology Institute, Academy of Military Medical Science.

**Cell medium:** DMEM supplemented with 10% FBS.

### 2. Condition

Virus was measured in grade 3rd laboratory of biosafety

### 3. Method

**CPE (cytopathic effect) assay of TCID$_{50}$:** 100$\mu$l of Vero $E_6$ cells were plated in 96-well plates at $2 \times 10^4$ cells per well. After 24 hr incubation at 37°C, Vero E6 monolayer cells were treated with 9 levels of SARS-associated coronavirus dilution by 10-fold dilution, 4 wells per dilution. The cells were incubated at 37°C and 5% $CO_2$. CPE (cytopathic effect) was examined daily by microscopy. CPE less than 25% was determined as +, 26-50% as ++, 51-75% as +++, 76-100% as ++++. CPE was recorded. Then TCID$_{50}$ was calculated by Reed-Muench method.

**Cytotoxicity of medicine:** Vero $E_6$ cells were inoculated into 96-well plates at $2 \times 10^4$ cells (100ul) per well. After 24-hr incubation at 37°C, cells grew up to monolayer. The medicine was diluted into 36, 18, 9, 4.5, 2.25$\mu$g /ml (final concentration) and added into wells each for 4 wells. The normal cells as control group were set. CPE of medicine group was daily observed during 5 -day period, and then the concentration of medicine exhibiting no toxicity was determined.

**CPE assay of the activity of the medicine against SARS-associated coronavirus:** 100$\mu$l of Vero $E_6$ cells were plated in 96-well plates at $2 \times 10^4$ cells per well. After 24hr incubation at 37°C, cells grew up to monolayer. The medicine at the maximal concentration exhibiting no cytotoxicity was diluted into 5 levels by 2-fold dilution and added

into wells (100$\mu$l per well). By incubation with 5% $CO_2$ at 37°C for 24-hour, different concentration of virus ($10^{-3}$, $10^{-4}$, $10^{-5}$) were added. After treatment with virus for 48-72 hours, CPE was examined (CPE less than 25% was determined as +, 26-50% as ++, 51-75% as +++, 76-100% as ++++, normal cell as -). The cells were divided into the normal group, the medicine control group, and the different dilution of virus control group, 4 wells per group. CPE was examined daily. Till cytopathic effect was obviously exhibited in the virus control group, the anti-virus activity of interferon was evaluated. The experiment was repeated. $IC_{50}$ of the medicine was calculated by Reed-Muench method.

### 4. Results

**Toxicity of virus:** $TCID_{50}$ of virus was $10^{-8}$.

**Cytotoxicity of medicine:** the concentration of Recombinant compound interferon exhibiting no cytotoxicity was 18$\mu$g/ml, the cells shape was similar with the control group, and no cytopathic effect was exhibited.

**The anti-virus effect of the medicine:** Shown in Table 9-A.1 and Table 9-A.2

**Table 9-A.1, the anti-virus effect of new-type recombinant compound interferon (first experiment)**

| Concentration of IFN ($\mu$g /ml) | CPE at different concentration of virus | | |
|---|---|---|---|
| | $10^{-3}$ | $10^{-4}$ | $10^{-5}$ |
| 18 | - | - | - |
| 9 | - | - | - |
| 4.5 | ++ | - | - |
| 2.25 | +++ | ++ | - |
| 1.125 | ++++ | ++++ | ++ |
| Virus control group | ++++ | ++++ | +++ |
| Normal group | - | - | - |
| Medicine control group | - | - | - |

**Table 9-A.2, the anti-virus effect of new-type recombinant compound interferon (second experiment)**

| Concentration of IFN ($\mu$g /ml) | CPE at different concentration of virus | | |
|---|---|---|---|
| | $10^{-3}$ | $10^{-4}$ | $10^{-5}$ |
| 18 | - | - | - |
| 9 | - | - | - |
| 4.5 | + | - | - |
| 2.25 | +++ | ++ | - |
| 1.125 | ++++ | ++++ | ++ |
| Virus control group | ++++ | ++++ | ++++ |
| Normal group | - | - | - |
| Medicine control group | - | - | - |

### 5. Conclusion

The concentration of the new-type recombinant compound interferon exhibiting no cytotoxicity at 18$\mu$g /ml. Its $IC_{50}$ were 1.27, 2.25, and 4.04$\mu$g/ml respectively according to the concentration of $10^{-5}$ (1000TCID50), $10^{-4}$(1000TCID50), $10^{-3}$(100000TCID50) of SARS-associated coronavirus (Table 9-A.3).

**Table 9-A.3, IC50 of IFN at different concentrations of virus**

| Dilution of virus | IC50 of IFN(ug/ml) |
|---|---|
| $10^{-3}$ | 4.04 |
| $10^{-4}$ | 2.25 |
| $10^{-5}$ | 1.27 |

**Principal:** Jin-yan Wang
**Laboratory assistant:** Yan-hong Zhao, Xiao-guang Ji, Xiao-yu Li.
**Original data:** Preserved in archives of Molecular Biology Department, microorganism and epidemiology Institute, Academy of Military Medical Science
**Date:** From May 12th to 30th, 2003

**EXAMPLE 9-B**

**In vitro effect of a new -type recombinant compound interferon and recombinant interferon -$\alpha$-2b injection on SARS-associated coronavirus**

**Sample supplied by:** Huiyang Life Engineering Ltd., Sichuan province

**Experimenter:** Molecular Biology Department, microorganism and epidemiology Institute, Academy of Military Medical Science

**Original data:** Preserved in muniment room of Molecular Biology Department, microorganism and epidemiology Institute, Academy of Military Medical Science

**1. Materials**

**Medicine:** New-type recombinant compound interferon , 618$\mu$g /ml , supplied by Huiyang Life Engineering Ltd., SiChuan Province; Anfulong (recombinant interferon -$\alpha$-2b injection), supplied by Hua-li-da Biology Engineering Ltd. Company, Tianjin City, 30ug/vial(300,OOOOIU/vial), Lot Number:20030105.
**Cells:** Vero $E_6$, supplied by Molecular Biology Department of Microorganism and Epidemiology Institute, Academy of Military Medical Science.
**Virus:** SARS-associated coronavirus, BJ-01, supplied by Molecular Biology Department of Microorganism and Epidemiology Institute, Academy of Military Medical Science.
**Condition:** Viruses were measured in grade 3$^{rd}$ laboratory of biosafety

**2. Method**

**$TCID_{50}$ was measured with CPE assay:** Vero $E_6$ cells were inoculated in 96-well plates at $2 \times 10^4$ cells (100$\mu$l) per well. After a 24-hr incubation at 37°C, Vero E6 monolayers were treated with 9 levels of SARS-associated coronavirus dilution by 10 times decreasing, each dilution per 4 wells. The cells were incubated at 37°C and 5% carbon dioxide. CPE was examined daily by phase-contrast microscopy. CPE less than 25% was determined as +, 26-50% as ++, 51-75% as +++, 76-100% as ++++. CPE was recorded. Then $TCID_{50}$ was calculated by Reed-Muench method.
**$TC_{50}$ of IFNs were measured by MTT assay:** Vero $E_6$ cells were inoculated in 96-well plates at $2 \times 10^4$ cells per well (100$\mu$l). After 24-hr incubation at 37°C, the supernatant liquid was removed when cells grew up to monolayer, then Vero $E_6$ was treated with different concentration of IFNs, each dilution per 4 wells. Normal group was set. After 5-day observation, the cells were mixed with MTT for 4 hours. After that, remove the liquid, and then thereafter DMSO were added into cells for 0.5 hour. The $OD_{570nm}$, was measured by microplate reader. Finally, $TC_{50}$ was

calculated by Reed-Muench method.

**The activity of the INFs against SARS-associated coronavirus was measured with MTT assay:** 100$\mu$l of Vero E$_6$ cells were inoculated in 96-well plates at $2\times10^4$ cells per well. After 24-hr incubation 37°C, cells became monolayer. The medicine dilution at the concentration of exhibiting no cytotoxicity was 5 times decreasing and there were 5 levels of dilution. Then each dilution was added to 4 wells, 100ul per well. After 24-hour incubation at 37°C and 5% $CO_2$, IFN solution was removed, then different concentrations of virus dilution (10000, 1000, 100 TCID$_{50}$) were added into dishes, 4 wells per dilution. The cells were divided into the normal group, the medicine control group, and the different dilution of virus control group (10000, 1000, 100 TCID$_{50}$). The cells were incubated at 37°C and 5% $CO_2$ for 48-72hr, until cytopathic effect was exhibited in the virus control group, CPE was recorded (CPE less than 25% was determined as +, 26-50% as ++, 51-75% as +++, 76-100% as ++++, normal cell as -). The growth ability of cells was measured with MTT assay, and then the antivirus effect of the INFs was evaluated. The experiment was repeated 3 times. IC$_{50}$ of the medicine was calculated by Reed-Muench method.

### 3. Results

**TCID$_{50}$ of virus:** TCID$_{50}$ of virus was $10^{-7}$.
**TC$_{50}$ of IFNs:** The concentration of new-type recombinant compound interferon exhibiting no cytotoxicity was 100$\mu$g/ml, and that of recombinant IFN-$\alpha$-2b was 12.5$\mu$g/ml, the cells shape was identical with the normal group at that concentration. TC50 of new-type recombinant compound interferon was 139.18$\mu$g/ml, that of recombinant IFN-$\alpha$-2b was 17.18$\mu$g/ml

**Table 9-B.1 TC$_{50}$ of IFNs**

| IFN | TC$_{50}$ ($\mu$g/ml) | | | Mean value (X$\pm$SD, n=3) |
|---|---|---|---|---|
| | 1st experiment | 2nd experiment | 3rd experiment | |
| new-type recombinant compound interferon | 141.42 | 125.96 | 150.08 | 139.18$\pm$12.22 |
| IFN-$\alpha$-2b | 17.68 | 15.75 | 18.10 | 17.18$\pm$1.25 |

**The anti-virus effect of the medicine**: The anti-virus effects of two IFNs were observed in vitro. The results of the experiments are shown on the Table 9-B.2, and the results of TI are shown on the Table 9-B.3.

**Table 9-B.2, The anti-virus activity of IFNs**

| IFNs | Concentration of virus (TCID$_{50}$) | IC$_{50}$ ($\mu$g/ml) | | | |
|---|---|---|---|---|---|
| | | 1st experiment | 2nd experiment | 3rd experiment | Mean value (X$\pm$SD, n=3 |
| new-type recombinant compound interferon | 10000 | 0.79 | 1.04 | 0.93 | 0.92$\pm$0.12 |
| IFN-$\alpha$-2b | | 5.04 | 4.56 | 4.65 | 4.75$\pm$0.25 |
| new-type recombinant compound interferon | 1000 | 0.19 | 0.18 | 0.18 | 0.18$\pm$0.01 |
| IFN-$\alpha$-2b | | 1.18 | 1.19 | 1.12 | 1.16$\pm$0.04 |
| new-type recombinant compound interferon | 100 | 0.08 | 0.10 | 0.11 | 0.10$\pm$0.02 |
| IFN-$\alpha$-2b | | 0.33 | 0.21 | 0.30 | 0.28$\pm$0.06 |

**Table 9-B.3, The anti-virus activity of IFNs**

| IFNs | Concentration of virus (TCID$_{50}$) | TC$_{50}$ ($\mu$g/ml) | IC$_{50}$ ($\mu$g/ml) | TI (TC$_{50}$/IC$_{50}$) |
|---|---|---|---|---|
| new-type recombinant compound interferon | 10000 | 139.18 | 0.92 | 151.28 |
| IFN-$\alpha$-2b | | 17.18 | 4.75 | 3.62 |
| new-type recombinant compound interferon | 1000 | 139.18 | 0.18 | 773.22 |
| IFN-$\alpha$-2b | | 17.18 | 1.16 | 14.78 |
| new-type recombinant compound interferon | 100 | 139.18 | 0.10 | 1391.80 |
| IFN-$\alpha$-2b | | 17.18 | 0.28 | 61.36 |

**4. Conclusion**

The protection effect of new-type recombinant compound interferon and IFN-$\alpha$-2b on Vero E$_6$ was observed in vitro, and the anti-virus ability of IFNs was manifested. IC$_{50}$ of new-type recombinant compound interferon on SARS-associated coronavirus at the concentration of 10000,1000,100 was 0.92,0.18,and 0.10$\mu$g/ml in three experiments, TI of that was 151.28, 773.32, and 1391.80 respectively. IC$_{50}$ of IFN-$\alpha$-2b was 4.75, 1.16, and 0.28$\mu$g/ml, TI (treatment index) of that was 3.62,14.78,61.36 respectively.

Most importantly, the two tests (See the above Examples 9A & 9B) of in vitro anti-SARS virus effect of rSIFN-co all testified that even the effective dose of rSIFN-co to inhibit SARS virus is 1/5 of that of Interferon $\alpha$-2b which was used clinically in China at present, the Treatment Index (TI) of rSIFN-co is nearly 50 times of that of Interferon $\alpha$-2b.(SEE: In vitro effect of a new-type recombinant compound interferon and recombinant interferon-$\alpha$2b injection on SARS-associated coronavirus. By The Institute of Microbiology & Epidemiology, Academy of Military Medical Science)

Thirty thousand sprays of rSIFN-co had been used among front-line nurses and doctors, and people at high risk in Sichuan province. The result shows that none of the nurses and doctors infected SARS in Sichuan Province.

**Principal**: Jin-yan Wang
**Laboratory assistant**: Yan-hong Zhao, Xiao-guang Ji, Min Zhang, Jing-hua, Zhao.
**Date**: From July 1st to 30th, 2003

**Example 10:**

**COMPARISON OF INHIBITORY EFFECTS OF DIFFERENT INTERFERONS ON HBV GENE EXPRESSION**

Hepatitis B virus (HBV) DNA contains consensus elements for transactivating proteins whose binding activity is regulated by interferons. Treatment of HBV-infected hepatocytes with interferons leads to inhibition of HBV gene expression. The aim of the present study was to characterize the effects of different interferons on HBV regulated transcription. Using transient transfection of human hepatoma cells with reporter plasmids containing the firefly luciferase gene under the control of HBV-Enhancer (EnH) I, Enh II and core promoter, Applicant studied the biological activities of three different interferons on transcription.

*Materials and Methods*

1. Interferons: IFN-con1 (Infergen®), IFN-Hui-Yang ($\gamma$SIFN-co) and IFN-beta 1b
2. Reporter plasmid: The DNA fragments containing HBV-Enhancer (EnH) I, Enh II and core promoter were prepared using PCR and blunt-end cloned into the Smal I site of the promoter- and enhancer-less firefly luciferase reporter plasmid pGL3-Basic (Promega, WI, USA). The resulting reporter plasmid was named as pGL3-HBV-Luc.
3. Cell Culture and DNA transfection: HepG2 cells were cultured in DMEM medium supplemented with 10% FBS and 100 U/ml penicillin and 100 ug/ml streptomycin. The cells were kept in 30°C, 5% CO2 incubator. The cells were transfected with pGL3-HBV-Luc reporter plasmid using Boehringer's Lipofectin transfection kit. After 18 hours, the medium containing transfection reagents was removed and fresh medium was added with or without interferons. The cells were kept in culture for another 48 hours.

4. Luciferase Assay: Forty-eight hours after addition of interferon, the cells were harvested and cell lysis were prepared. The protein concentration of cell lysates were measured using Bio-Rad Protein Assay kit. The luciferase activity was measured using Promega's Luciferase Reporter Assay Systems according to the instructions of manufacturer.

*RESULTS*

Expression of Luciferase Activity in Different Interferon - Treated Cell Lysates

| No treatment | IFN-con1 | IFN-Hui-Yang | IFN-beta 1b |
|---|---|---|---|
| 100 | 48+8 | 29+6 | 64+10 |

This result shows that γSIFN-co inhibits most effectively on the expression of HBV gene expression.

**Example 11:**

**SIDE EFFECTS AND CHANGES IN BODY TEMPERATURE WHEN USING γSIFN-co**

There are usually more side effects to using interferon. The side effects includes: nausea, muscle soreness, loss of appetite, hair loss, hypoleucocytosis (hypoleukmia; hypoleukocytosis; hypoleukia), and decrease in blood platelet, etc.

*METHOD*

Sample patients are divided into two groups. 11 patients in Group A were injected with 9μg Infergen®. 10 patients in Group B were injected with 9μg γSIFN-co. Both groups were monitored for 48 hours after injections. First monitoring was recorded 1 hour after injection. After that, records were taken every 2 hours.

Table 11.1 is the comparison of side effects between patients being injected with 9μg of Infergen® and 9μg of γSIFN-co.

Table 11.1. Side Effects

| | | γSIFN-co 9μg | Infergen® 9μg |
|---|---|---|---|
| | | Person: n=10 | Person: n=11 |
| Body Systems | Reactions | Headcount | Headcount |
| In General | Feeble | 3 | 3 |
| | Sole heat | 1 | |
| | frigolabile | 3 | 4 |
| | Leg strengthless | | 3 |
| | Mild lumbago | 2 | 1 |
| | Body soreness | 4 | 5 |
| Central Nervous System/ Peripheral Nervous System | Headache | 3 | 6 |
| | Dizziness | 2 | 11 |
| | Drowsiness | | 3 |
| Gastroenterostomy | Apoclesis | 1 | |
| | Celiodynia | 1 | |
| | Diarrhea | 1 | |

(continued)

|  |  | γSIFN-co 9μg | Infergen® 9μg |
|---|---|---|---|
| Musculoskeletal system | Myalgia | 1 | 2 |
|  | Arthralgia | 2 |  |
| Respiratory system | Stuffy nose | 1 |  |
| Paropsia | Swollen Eyes |  | 1 |

RESULTS

For those patients who were injected with ySIFN-co, the side effects were minor. They had some common symptoms similar to flu, such as: headache, feebleness, frigolability, muscle soreness, hidrosis, arthralgia (arthrodynia; arthronalgia). The side effects of those patients whom were injected with Infergen® were worse than those injected with γSIFN-co.

From Figures 9A-1, 9A-2, 9B-1, and 9B-2, it was obvious that the body temperatures of sample patients in Group A were higher than the patients in Group B. It also reflected that the endurance of γSIFN-co was much better than Infergen®.

**Example 12:**

**CRYSTAL GROWTH of γSIFN-co AND TEST OF CRYSTALLOGRAPHY PARAMETER**

Crystal of γSIFN-co. Two types of crystal were found after systematically trial and experiment. (See Figures 10-12)

1. Crystal Growth

Dissolve γSIFN-co protein with pure water ($H_2O$) to 3mg/ml in density. Search of crystallization by using Hampton Research Crystal Screen I and II which was made by Hampton Company. By using Drop Suspension Diffusion Method, liquid 500μl, drop 1 μl protein + 1 μl liquid, in 293K temperature. First 2 different types of small crystals were found as listed in Table 12.1.

**Table 12.1. Screen of γSIFN-co Crystallin**

| Condition | I | II |
|---|---|---|
| Diluent | 0.1M Tris-HCl PH=8.75 | 0.1M HEPES PH=7.13 |
| Precipitant | 17.5% (w/v) PEG550 MME | 10% (w/v) PEG6K |
| Additives | 0.1M NaCl | 3% (v/v) MPD |
| Temperature | 293K | 293K |
| Crystal Size (mm) | 0.2x0.2x0.1 | 0.6x0.02x0.02 |
| Crystallogram | Figure 10 | Figure 11 |

2. Data Collection and Processing

Crystal I was used to collect X-Ray diffraction data and preliminary analysis of crystallography. Parameters were also tested. The diffraction data was collected under room temperature. Crystal I (Condition I) was inserted into a thin siliconized wall tube. Using BrukerAXS Smart CCD detector, the light source is CuKα (λ=1.5418Å) generated by Nonius FR591 X-ray generator. Light power 2000 KW (40 kv x 50mA), wave length 1.00Å, under explosion 60 second, Δφ=2°, the distance between crystal and detector was 50mm. Data was processed for using Proteum Procedure Package by Bruker Company. See Figure 12 for crystal diffraction pattern (partially). See Table 12.2 for the result of the process.

**Table 12.2. Results of Crystallography Parameters Parameters**

| a (Å) | 82.67 |
|---|---|
| b (Å) | 108.04 |

(continued)

| | |
|---|---|
| c (Å) | 135.01 |
| α (°) | 90.00 |
| β (°) | 90.00 |
| γ (°) | 98.35 |

Space Group P2 or P2$_1$
Sharpness of separation 5 Å
Asymmetric molecule # 10
Dissolution 57.6%

Besides, there was no crystal growth of γSIFN-co based on previous publications. The closest result to the γSIFN-co was huIFN-a2b but the screen was very complicated. After seeding 3 times, crystal grew to 0.5x0.5x0.3mm, sharpness of separation was 2.9 Å, space group was P2$_1$. The crystals were also big, asymmetric molecule number was 6, and dissolution was about 60%.

**Claims**

1. A recombinant interferon obtainable by a process comprising introducing into E. coli the polynucleotide sequence shown in Figure 1, for use as a medicament for treating a viral disease in a subject.

2. A pharmaceutical composition comprising a recombinant interferon obtainable by a process comprising introducing into E. coli the polynucleotide sequence shown in Figure 1 and a pharmaceutically acceptable carrier, for use in the treatment of a viral disease.

3. The recombinant interferon for use of claim 1 or the pharmaceutical composition for use of claim 2, wherein the viral disease is hepatitis A, hepatitis B, hepatitis C, other types of hepatitis, or virus-induced upper respiratory disease.

4. The recombinant interferon for use of claim 1 or the pharmaceutical composition for use of claim 2, wherein the viral disease is an infection caused by Epstein-Barr virus, Cytomegalovirus, herpes simplex virus, other types of herpes virus, papovavirus, poxvirus, picornavirus, adenovirus, rhinovirus, human T-cell leukemia virus I, human T-cell leukemia virus II, human T-cell leukemia virus III, or avian influenza virus.

5. A recombinant interferon obtainable by a process comprising introducing into E. coli the polynucleotide sequence shown in Figure 1, for use as a medicament for treating tumor in a subject.

6. A pharmaceutical composition comprising a recombinant interferon obtainable by a process comprising introducing into E. coli the polynucleotide sequence shown in Figure 1 and a pharmaceutically acceptable carrier, for use in the treatment of a tumor.

7. The recombinant interferon for use of claim 5 or the pharmaceutical composition for use of claim 6, wherein the tumor is skin cancer, basal cell carcinoma, malignant melanoma, liver cancer, renal cell carcinoma, thyroid cancer, rhinopharyngeal cancer, solid carcinoma, prostate cancer, stomach cancer, abdominal cancer, esophageal cancer, rectal cancer, pancreatic cancer, breast cancer, ovarian cancer, superficial bladder cancer, hemangioma, cervical cancer, non-small cell lung cancer, small cell lung cancer, glioma, acute leucocythemia, chronic leucocythemia, chronic myelocytic leukemia, hairy cell leukemia, lymphadenoma, multiple myeloma, polycythemia vera, or Kaposi's sarcoma.

8. The recombinant interferon for use of claim 1 or 5, wherein the interferon is administered via oral administration, vein injection, muscle injection, peritoneal injection, subcutaneous injection, nasal administration, mucosal administration, or by inhalation.

9. The recombinant interferon for use of claim 1 or 5, wherein the recombinant interferon is formulated as tablet, capsule, oral liquid, paste, injection, spray, suppository, or solution.

**Patentansprüche**

1.  Rekombinantes Interferon, das durch einen Prozess erhalten werden kann, der das Einführen der in Figur 1 gezeigten Polynukleotidsequenz in E. coli umfasst, zur Verwendung als Medikament zum Behandeln einer viralen Krankheit in einem Individuum.

2.  Pharmazeutische Zusammensetzung, umfassend ein rekombinantes Interferon, das durch einen Prozess erhalten werden kann, der das Einführen der in Figur 1 gezeigten Polynukleotidsequenz in E. coli umfasst, und einen pharmazeutisch zulässigen Träger zur Verwendung bei der Behandlung einer viralen Krankheit.

3.  Rekombinantes Interferon zur Verwendung nach Anspruch 1 oder die pharmazeutische Zusammensetzung zur Verwendung nach Anspruch 2, wobei es sich bei der viralen Krankheit um Hepatitis A, Hepatitis B, Hepatitis C, andere Formen von Hepatitis oder eine virusinduzierte Krankheit der oberen Atemwege handelt.

4.  Rekombinantes Interferon zur Verwendung nach Anspruch 1 oder die pharmazeutische Zusammensetzung zur Verwendung nach Anspruch 2, wobei es sich bei der viralen Krankheit um eine Infektion handelt, die vom Epstein-Barr-Virus, Zytomegalovirus, Herpes-simplex-Virus, anderen Arten des Herpes-Virus, Papovavirus, Poxvirus, Pikornavirus, Adenovirus, Rhinovirus, dem humanen T-Zell-Leukämie-Virus I, dem humanen T-Zell-Leukämie-Virus II, dem humanen T-Zell-Leukämie-Virus III oder einem aviären Influenzavirus verursacht wird.

5.  Rekombinantes Interferon, das durch einen Prozess erhalten werden kann, der das Einführen der in Figur 1 gezeigten Polynukleotidsequenz in E. coli umfasst, zur Verwendung als Medikament zum Behandeln eines Tumors in einem Individuum.

6.  Pharmazeutische Zusammensetzung, umfassend ein rekombinantes Interferon, das durch einen Prozess erhalten werden kann, der das Einführen der in Figur 1 gezeigten Polynukleotidsequenz in E. coli umfasst, und einen pharmazeutisch zulässigen Träger zur Verwendung bei der Behandlung eines Tumors.

7.  Rekombinantes Interferon zur Verwendung nach Anspruch 5 oder die pharmazeutische Zusammensetzung zur Verwendung nach Anspruch 6, wobei es sich bei dem Tumor um Hautkrebs, ein Basalzellkarzinom, ein malignes Melanom, Leberkrebs, ein Nierenzellkarzinom, Schilddrüsenkrebs, rhinopharyngealen Krebs, ein solides Karzinom, Prostatakrebs, Magenkrebs, abdominalen Krebs, ösophagealen Krebs, rektalen Krebs, Bauchspeicheldrüsenkrebs, Brustkrebs, Ovarialkrebs, oberflächlichen Blasenkrebs, ein Hämangiom, Gebärmutterhalskrebs, nicht-kleinzelligen Lungenkrebs, kleinzelligen Lungenkrebs, ein Gliom, akute Leukozythämie, chronische Leukozythämie, chronische myeloische Leukämie, Haarzellenleukämie, ein Lymphadenom, ein multiples Myelom, Polycythaemia vera oder Kaposi-Sarkom handelt.

8.  Rekombinantes Interferon zur Verwendung nach Anspruch 1 oder 5, wobei das Interferon durch orale Verabreichung, Veneninjektion, Muskelinjektion, peritoneale Injektion, subkutane Injektion, nasale Verabreichung, mukosale Verabreichung oder durch Inhalation verabreicht wird.

9.  Rekombinantes Interferon zur Verwendung nach Anspruch 1 oder 5, wobei das rekombinante Interferon als Tablette, Kapsel, orale Flüssigkeit, Paste, Injektion, Spray, Zäpfchen oder Lösung formuliert ist.

**Revendications**

1.  Interféron recombinant pouvant être obtenu par un processus comprenant l'introduction dans E. coli de la séquence polynucléotidique illustrée sur la figure 1, pour son utilisation en tant que médicament pour le traitement d'une maladie virale chez un sujet.

2.  Composition pharmaceutique comprenant un interféron recombinant pouvant être obtenu par un processus comprenant l'introduction dans *E. coli* de la séquence polynucléotidique illustrée sur la figure 1 et un vecteur pharmaceutiquement acceptable, pour son utilisation dans le traitement d'une maladie virale.

3.  Interféron recombinant pour son utilisation selon la revendication 1, ou la composition pharmaceutique pour son utilisation selon la revendication 2, dans lequel la maladie virale est l'hépatite A, l'hépatite B, l'hépatite C, d'autres types d'hépatites, ou une maladie des voies respiratoires supérieures induite par un virus.

**4.** Interféron recombinant pour son utilisation selon la revendication 1, ou la composition pharmaceutique pour son utilisation selon la revendication 2, dans lequel la maladie virale est une infection provoquée par le virus d'Epstein-Barr, un Cytomégalovirus, le virus de l'herpès simplex, d'autres types de virus de l'herpès, un papovavirus, un poxvirus, un picornavirus, un adénovirus, un rhinovirus, le virus de la leucémie humaine à cellules T de type I, le virus de la leucémie humaine à cellules T de type II, le virus de la leucémie humaine à cellules T de type III, ou un virus de la grippe aviaire.

**5.** Interféron recombinant pouvant être obtenu par un processus comprenant l'introduction dans E. coli de la séquence polynucléotidique illustrée sur la figure 1, pour son utilisation en tant que médicament pour le traitement d'une tumeur chez un sujet.

**6.** Composition pharmaceutique comprenant un interféron recombinant pouvant être obtenu par un processus comprenant l'introduction dans *E. coli* de la séquence polynucléotidique illustrée sur la figure 1 et un vecteur pharmaceutiquement acceptable, pour son utilisation dans le traitement d'une tumeur.

**7.** Interféron recombinant pour son utilisation selon la revendication 5 ou la composition pharmaceutique pour son utilisation selon la revendication 6, dans lequel la tumeur est le cancer de la peau, le carcinome des cellules basales, le mélanome malin, le cancer du foie, le carcinome des cellules rénales, le cancer de la thyroïde, le cancer du rhinopharynx, le carcinome solide, le cancer de la prostate, le cancer de l'estomac, le cancer de l'abdomen, le cancer de l'oesophage, le cancer rectal, le cancer du pancréas, le cancer du sein, le cancer de l'ovaire, le cancer superficiel de la vessie, l'hémangiome, le cancer du col de l'utérus, le cancer du poumon non à petites cellules, le cancer du poumon à petites cellules, le gliome, la leucocythémie aiguë, la leucocythémie chronique, la leucémie myélogène chronique, la leucémie à tricholeucocytes, le lymphadénome, le myélome multiple, la polycythémie vraie ou le sarcome de Kaposi.

**8.** Interféron recombinant pour son utilisation selon la revendication 1 ou la revendication 5, dans lequel l'interféron est administré par voie orale, par injection intraveineuse, par injection intramusculaire, par injection intrapéritonéale, par injection sous-cutanée, par voie nasale, par voie muqueuse ou par inhalation.

**9.** Interféron recombinant pour son utilisation selon la revendication 1 ou la revendication 5, dans lequel l'interféron recombinant est formulé en un comprimé, en une capsule, en un liquide pour administration orale, en une pâte, en une injection, en une pulvérisation, en un suppositoire, ou en une solution.

## Figure 1

```
5'            11        21        31        41        51
+1 M  C  D  L  P  Q  T  H  S  L  G  N  R  R  A  L  I  L  L  A
   1 ATGTGCGACC TGCCGCAGAC CCACTCCCTG GGTAACCGTC GTGCTCTGAT CCTGCTGGCT
     TACACGCTGG ACGGCGTCTG GGTGAGGGAC CCATTGGCAG CACGAGACTA GGACGACCGA


5'            71        81        91        101       111
+1 Q  M  R  R  I  S  P  F  S  C  L  K  D  R  H  D  F  G  F  P
  61 CAGATGCGTC GTATCTCCCC GTTCTCCTGC CTGAAAGACC GTCACGACTT CGGTTTCCCG
     GTCTACGCAG CATAGAGGGG CAAGAGGACG GACTTTCTGG CAGTGCTGAA GCCAAAGGGC


5'           131       141       151       161       171
+1 Q  E  E  F  D  G  N  Q  F  Q  K  A  Q  A  I  S  V  L  H  E
 121 CAGGAAGAAT TCGACGGTAA CCAGTTCCAG AAAGCTCAGG CTATCTCCGT TCTGCACGAA
     GTCCTTCTTA AGCTGCCATT GGTCAAGGTC TTTCGAGTCC GATAGAGGCA AGACGTGCTT


5'           191       201       211       221       231
+1 M  I  Q  Q  T  F  N  L  F  S  T  K  D  S  S  A  A  W  D  E
 181 ATGATCCAGC AGACCTTCAA CCTGTTCTCC ACCAAAGACT CCTCCGCTGC TTGGGACGAA
     TACTAGGTCG TCTGGAAGTT GGACAAGAGG TGGTTTCTGA GGAGGCGACG AACCCTGCTT


5'           251       261       271       281       291
+1 S  L  L  E  K  F  Y  T  E  L  Y  Q  Q  L  N  D  L  E  A  C
 241 TCCCTGCTGG AAAAATTCTA CACCGAACTG TACCAGCAGG TGAACGACCT GGAAGCTTGC
     AGGGACGACC TTTTTAAGAT GTGGCTTGAC ATGGTCGTCG ACTTGCTGGA CCTTCGAACG


5'           311       321       331       341       351
+1 V  I  Q  E  V  G  V  E  E  T  P  L  M  N  V  D  S  I  L  A
 301 GTTATCCAGG AAGTTGGTGT TGAAGAAACC CCGCTGATGA ACGTTGACTC CATCCTGGCT
     CAATAGGTCC TTCAACCACA ACTTCTTTGG GGCGACTACT TGCAACTGAG GTAGGACCGA


5'           371       381       391       401       411
+1 V  K  K  Y  F  Q  R  I  T  L  Y  L  T  E  K  K  Y  S  P  C
 361 GTTAAAAAAT ACTTCCAGCG TATCACCCTG TACCTGACCG AAAAAAAATA CTCCCCGTGC
     CAATTTTTTA TGAAGGTCGC ATAGTGGGAC ATGGACTGGC TTTTTTTTAT GAGGGGCACG


5'           431       441       451       461       471
+1 A  W  E  V  V  R  A  E  I  M  R  S  F  S  L  S  T  N  L  Q
 421 GCTTGGGAAG TTGTTCGTGC TGAAATGATG CGTTCCTTCT CCCTGTCCAC CAACCTGCAG
     CGAACCCTTC AACAAGCACG ACTTTAGTAC GCAAGGAAGA GGGACAGGTG GTTGGACGTC


5'           491       501
+1 E  R  L  R  R  K  E  #
 481 GAACGTCTGC GTCGTAAAGA ATAA
     CTTGCAGACG CAGCATTTCT TATT
```

Figure 2

```
5'        11         21         31         41         51
+1  M  C  D    L  P  Q  T    H  S  L    G  N  R    R  A  L  I    L  L  A
 1 ATGTGTGATT TACCTCAAAC TCATTCTCTT GGTAACCGTC GCGCTCTCAT TCTGCTGGCA
   TACACACTAA ATGGAGTTTG AGTAAGAGAA CCATTGGCAG CGCGAGACTA AGACGACCGT


5'        71         81         91          1         11
+1  Q  N  R    R  I  S  P    F  S  C    L  K  D    R  H  D  F    G  F  P
61 CAGATGCGTG GTATTTCCCC GTTTAGCTGC CTGAAAGACC GTCACGACTT CGGCTTTCCG
   GTCTACGCAC CATAAAGGGG CAAATCGACG GACTTTCTGG CAGTGCTGAA GCCGAAAGGC


5'        31         41         51         61         71
+1  Q  E  E    F  D  G  N    Q  F  Q    K  A  Q    A  I  S  V    L  H  E
121 CAAGAAGAGT TCGATGGCAA CCAATTCCAG AAAGCTCAGG CAATCTCTGT ACTGCACGAA
    GTTCTTCTCA AGCTACCGTT GGTTAAGGTC TTTCGAGTCC GTTAGAGACA TGACGTGCTT


5'        91          1         11          21        31
+1  M  I  Q    Q  T  F  N    L  F  S    T  K  D    S  S  A  A    W  D  E
181 ATGATCCAAC AGACCTTCAA CCTGTTTTCC ACTAAAGACA GCTCTGCTGC TTGGGACGAA
    TACTAGGTTG TCTGGAAGTT GGACAAAAGG TGATTTCTGT CGAGACGACG AACCCTGCTT


5'        51         61         71         81         91
+1  S  L  L    E  K  F  Y    T  E  L    Y  Q  Q    L  N  D  L    E  A  C
241 AGCTTGCTGG AGAAGTTCTA CACTGAACTG TATCAGCAGC TGAACGACCT GGAAGCATGC
    TCGAACGACC TCTTCAAGAT GTGACTTGAC ATAGTCGTCG ACTTGCTGGA CCTTCGTACG


5'        11         21         31         41         51
+1  V  I  Q    E  V  G  V    E  E  T    P  L  M    N  V  D  S    I  L  A
301 GTAATCCAGG AAGTTGGTGT AGAAGAGACT CCGCTGATGA ACGTCGACTC TATTCTGGCA
    CATTAGGTCC TTCAACCACA TCTTCTCTGA GGCGACTACT TGCAGCTGAG ATAAGACCGT
```

## Figure 3

Figure 4

Figure 5

Figure 5 A

Figure 6-A

Figure 6-B

Figure 6-C

Figure 6-D

Figure 7A

Figure 7B

Figure 7C

Figure 7D

1. Pre-spray

Figure B

Figure 9A-1

Figure 9A-2

Figure 9B-1

Figure 9B-2

Figure 10

Figure 11

Figure 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4695623 A
- US 4897471 A
- US 5372808 A
- CN 97193506
- CN 98114663

**Non-patent literature cited in the description**

- Scientists Rush to Create Vaccine for Bird Flu - Just in Case. *The Wall Street Journal,* 28 January 2004
- *Journal of Interferon and Cytokine Research,* 1996, vol. 16, 489-499
- **JOSEPH SAMBROOK ; DAVID W. RUSSELL.** Molecular Cloning: A laboratory Manual. Cold Spring Harbor Laboratory Press, December 2000
- **BLATT LM ; DAVIS JM ; KLEIN SB et al.** The biologic activity and molecular characterization of a novel synthetic interferon-alpha species, consensus interferon. *Journal of Interferon and Cytokine Research,* 1996, vol. 16 (7), 489-499
- Production characterization and biological effects of recombinant DNA derived human IFN-$\alpha$ and IFN-$\gamma$ analogs. **ALTON,K. et al.** The Biology of Interferon System. Elsevier Science Publishers, 1983, 119-128
- **PFEFFER LM.** Biologic activity of natural and synthetic type 1 interferons. *Seminars in Oncology,* 1997, vol. 24 (3), S9-63, S9-69
- **OZES ON ; REITER Z ; KLEIN S et al.** A comparison of interferon-con1 with natural recombinant interferons-(: antiviral, antiproliferative, and natural killer-inducing activities. *J. Interferon Res.,* 1992, vol. 12, 55-59
- **HEATHCOTE EJL ; KEEFFE EB ; LEE SS et al.** Re-treatment of chronic hepatitis C with consensus interferon. *Hepatology,* 1998, vol. 27 (4), 1136-1143
- **KLEIN ML ; BARTLEY TD ; LAI PH et al.** Structural characterization of recombinant consensus interferon-alpha. *Journal of Chromatography,* 1988, vol. 454, 205-215
- The Wisconsin Package. Genetics Computer Group, Inc, 1992
- **NISHIMURA, A et al.** A rapid and highly efficient method for preparation of competent E. coli cells. *Nuclei. Acids Res.,* 1990, vol. 18, 6169
- **SAMBROOK, J. ; E. F. FRITSCH ; T. MANIATIS.** Molecular Cloning:A laboratory manual. CSH Laboratory Press, 1989